# EUROPÄISCHE PATENTSCHRIFT

(11) **EP 2 988 835 B1**
(45) Veröffentlichungstag und Bekanntmachung des Hinweises auf die Patenterteilung: **17.11.2021**
(21) Anmeldenummer: 14726324.8
(22) Anmeldetag: 25.04.2014
(51) Int. Cl.: A63B 23/02, A63B 22/16, A63B 24/00, A61H 1/00, A63B 26/00, A63B 22/00, A63B 21/005, A63B 69/00, A63B 23/035, A61H 23/02, A61B 5/11, A63B 21/008, A63B 21/00, A61B 5/107, A63B 21/072

(54) **VORRICHTUNG FÜR EIN TRAINING UND/ODER EINE ANALYSE DES BEWEGUNGSAPPARATS EINES BENUTZERS**
APPARATUS FOR TRAINING AND/OR ANALYSIS OF THE MUSCULOSKELETAL SYSTEM OF A USER
DISPOSITIF D'EXERCICE ET/OU D'ANALYSE DE L'APPAREIL LOCOMOTEUR D'UN UTILISATEUR

(30) Priorität: 25.04.2013 DE 102013007131; 25.04.2013 DE 202013003859 U; 14.11.2013 DE 202013010263 U
(43) Veröffentlichungstag der Anmeldung: 02.03.2016
(73) Patentinhaber: Schiessl, Hans, 75177 Pforzheim (DE)
(72) Erfinder: Schiessl, Hans, 75177 Pforzheim (DE)
(74) Vertreter: Leitner, Waldemar
(86) Internationale Anmeldenummer: PCT/EP2014/001108
(87) Internationale Veröffentlichungsnummer: WO 2014/173545

(56) Entgegenhaltungen:
- EP-A1- 2 160 168
- FR-A1- 2 809 615
- JP-A- 2004 261 256
- US-A- 5 176 598
- US-A- 5 203 321
- US-A- 5 474 520
- US-A1- 2005 251 067
- US-A1- 2008 161 169
- US-A1- 2011 256 983
- US-B1- 6 878 102

## Beschreibung

Die vorliegende Erfindung betrifft eine Vorrichtung für ein Training und/oder eine Analyse eines Bewegungsapparats eines Benutzers, wobei die Vorrichtung mindestens ein Grundelement, das durch eine Antriebseinrichtung in periodische und/oder aperiodische Bewegungen zur Erzeugung einer auf den Benutzer einwirkenden Anregungsfunktion versetzbar ist, aufweist, wobei das derart angetriebene Grundelement einen oder beide der auf ihm aufsetzenden Füße in seiner bzw. ihrer Gesamtheit periodisch anhebt und absenkt.

Aus der EP 2 160 168 der Anmelderin ist eine Vorrichtung für ein Training und/oder eine Analyse eines Bewegungsapparates eines Benutzers bekannt, wobei die Vorrichtung mindestens ein Grundelement, das durch eine Antriebseinrichtung in periodische und/oder aperiodische Bewegungen zur Erzeugung einer auf den Benutzer einwirkenden Anregungsfunktion versetzbar ist. Die Vorrichtung besitzt einen Sockel mit einem Ausleger, an dem um eine Drehachse drehbar gelagertes Grundelement angeordnet ist, welches als Grundplatte ausgeführt ist. Das als Wippe fungierende Grundelement wird von einer Antriebseinrichtung angetrieben, welche dazu dient, das Grundelement in Schwingungen zu versetzen. Die Antriebseinrichtung weist einen Elektromotor auf, welcher über einen Riemen zwei um weitere Achsen drehbar gelagerte Antriebsscheiben antreibt. An einem ersten Anlenkpunkt der Antriebsscheiben ist jeweils ein erstes Ende eines Pleuels angelenkt und ein zweites Ende desselben greift über einen weiteren Anlenkpunkt an der Grundplatte an, so dass durch eine Drehbewegung der Antriebsscheiben die Grundplatte in sinusförmige Schwingungen versetzt wird. Die Vorrichtung weist einen Sensor zur Ermittlung einer Antwortfunktion des Benutzers der Vorrichtung auf die durch das Grundelement auf ihn aufgeprägte Anregungsfunktion auf, dessen Sensorsignale einer Auswerteeinrichtung der Vorrichtung zugeführt sind.

Eine weitere derartige Vorrichtung ist aus der EP 0 929 284 der Anmelderin bekannt. Das in dieser Druckschrift beschriebene Gerät zur Stimulation von Muskeln des Bewegungsapparates, insbesondere im Bein- und Rückenbereich, weist ein an einem Gestell angeordnetes Grundelement in Form einer Wippe auf, die über einen Antriebsmechanismus um eine horizontale Schwenkachse oszillierend verschwenkbar ist und deren über die Schwenkachse überstehender Ausleger mit je einer Trittfläche versehen ist. Die Wippe ist mit einer Schwenkachse an gestellfesten Lagerblöcken gelagert und jeden Wippenausleger ist ein durch einen gemeinsamen Elektromotor oszillierend antreibbarer Hubmechanismus zugeordnet.

Diesen bekannten Vorrichtungen liegt die Erkenntnis zugrunde, dass eine therapeutisch wirksame Stimulation von Muskelfasern nur dann möglich ist, wenn die Muskelfasern von einem konstitutionsbedingten Grundtonus aus in einem definierten Schwellenbereich oszillierend stimuliert werden. Der Grundtonus wird naturgemäß durch das auf der Beinmuskulatur lastende Eigengewicht eingestellt, während die periodische Stimulanz durch einen geeigneten Antriebsmechanismus in die Muskulatur eingeleitet werden kann. Wenn sich ein Patient mit seinen Fußballen auf die beiden Trittflächen auf den Wippenauslegern stellt, so kann er durch mehr oder weniger tiefe Kniebeugen in seiner Muskulatur einen Grundtonus einstellen, dem die Oszillation der Wippenausleger überlagert wird. Durch die gegensinnige Oszillation der Wippenausleger wird die periodische Bewegung über die Gelenke und unter regelnder Einwirkung des vegetativen Nervensystems abgefangen, ohne dass es zu einer Verschiebung des Körperschwerpunkts kommt.

Aus der DE 196 34 396 ist ein Gerät zur Stimulation des Bewegungsapparates und der Muskulatur, insbesondere im Bein- und Rückbereich bekannt, bei dem vorgesehen ist, dass an einem Gestell zwei Trittflächen angeordnet sind, die mittels eines Antriebsmechanismus im Gegentakt oszillierend heb- und senkbar sind. Der Grundtonus der Muskulatur wird durch das Eigengewicht des auf den Trittflächen stehenden Patienten eingestellt, während die Muskelstimulation durch die oszillierende Trittflächenbewegung erfolgt.

Aus der US 2006/0229159 A1 ist ein Balance-Trainingsgerät bekannt, welches dazu dient, die Balancier-Fähigkeit eines Benutzers zu trainieren. Dieses Trainingsgerät weist eine Grundplatte auf, die von einem Motor angetrieben wird. Ein Drehwinkelsensor dient zur Erfassung des Drehwinkels der Grundplatte. Des weiteren ist eine Drehmomentmesseinrichtung vorgesehen, die dazu dient, das vom Benutzer auf die Grundplatte aufgebrachte Drehmoment zu messen. Die Ausgangssignale des Drehwinkelsensors und der Drehmomentmesseinrichtung werden einer Auswerteeinrichtung zugeführt, deren Ausgangssignal zu einem Motor-Controller geleitet wird, der dann entsprechende Steuersignale für den Motor erzeugt. Hierzu wird in der Auswerteeinrichtung entsprechend eines vorgegebenen kinetischen Modells, welches die mechanischen Charakteristiken der Grundplatte vorgibt, die entsprechende Kompensationsdrehbewegung der Grundplatte berechnet und den Motor entsprechend geregelt.

Es erfolgt also eine Drehbewegung der Platte als Reaktion auf die vom Benutzer ausgelöste Drehmomentbeaufschlagung, die Auswerteeinheit fungiert hier als Regeleinrichtung, welche das Drehmoment des Motors entsprechend einem kinetischen Modell regelt. In das kinetische Modell gehen als virtuelle Parameter das Trägheitsmoment der Grundplatte, deren Federkonstante und deren Dämpfung ein, so dass durch eine entsprechende Auswahl einer oder mehrerer der vorgenannten virtuellen Parameter unterschiedlich träge, federnde und/oder dämpfende Grundplatten simuliert und derart unterschiedliche Trainingsbedingungen und somit Balance-Anforderungen vorgegeben werden können. Das vom Benutzer vorgesehene Drehmoment wird dadurch gemäß dem vorgegebenen kinetischen Modell und den bei dieses eingehenden virtuellen Parametern durch den Motor kompensiert. Die bekannte Vorrichtung erlaubt somit lediglich durch die Simulation unterschiedlicher Grundplatten die Bereitstellung von unterschiedlichen Trainingsbedingungen, ist aber nicht in der Lage, Aufschlüsse über Parameter des Bewegungsapparates des Benutzers zu geben. Somit wird insbesondere nicht der zeitliche Zusammenhang der Verläufe der Messgrößen berücksichtigt.

Die DE 20 2005 002 086 beschreibt eine Vibrationstrainingsgerät, welches ein Gehäuse mit einer Schwingungsplattform und einen Schwingungsgenerator aufweist, der mit der Schwingungsplattform verbunden ist, wobei ein Steuergerät den Schwingungsgenerator regelt. Eine Schwingungssensoreinheit ist am Körper des Benutzers befestigbar und die am Körper aufgenommenen Schwingungen werden in Form von Signalen an das Steuergerät übertragen. Dieses regelt dann den Schwingungsgenerator in Anpassung an die Körperschwingungen. Indem durch diesen Regelkreis die aufgenommenen Körperschwingungen mit voreingestellten Grenzwerten kontinuierlich verglichen werden und eine Regelung durch das Steuergerät vorliegt, wird erreicht, dass die über die Schwingungsplattform in den Körper eingeleiteten Vibrationen keine schädliche Wirkung entfalten, da beim Überschreiten dieser Grenzwerte das Steuergerät mittels einer Abschaltautomatik den Schwingungsgenerator heruntersetzt oder ihn sogar stillsetzt.

Die DE 611 11 016 T2 beschreibt eine automatische Vorrichtung zur optimierten Muskelstimulierung wobei die Stimulierung für periodische Kontraktionen eines oder mehrerer Muskel eines Benutzers mit konstanter Frequenz sorgt. Hierzu sind ein oder mehrere Detektoren vorgesehen, die jeweils auf einen entsprechend zu stimulierenden Muskeln des Benutzers aufgebracht werden. Dann wird eine optimale Frequenz der periodischen Muskelkontraktion innerhalb eines Bereichs zwischen einer unteren Grenzfrequenz und einer oberen Grenzfrequenz aufgrund entsprechender periodischer mechanischer Stimulierung durch mechanische Mittel vorbestimmt, entsprechend welcher die Summe der Amplituden der Signale, die von den Detektoren von den entsprechend stimulierten Muskeln als Reaktion auf die Stimulierung geliefert werden, die größte ist, und die mechanischen Mittel erzeugen dann eine periodische Kontraktion der Muskeln mit der vorbestimmten optimalen Frequenz. Dieses Gerät sieht also vor, dass mittels Sensoren eine optimale Stimulierungsfrequenz der Muskeln bestimmt wird und dann die mechanischen Mitteln dementsprechend justiert werden.

Die DE 198 46 982 beschreibt ein Verfahren und ein System zur Überwachung der Haltung eines Benutzers an einem Trainingsgerät, wobei am Körper des Benutzers und/oder an bestimmten Stellen des Trainingsgeräts Sensoren angebracht werden. Die von den Sensoren erfassten Messdaten werden in einer Auswerteeinrichtung analysiert, um eine fehlerhafte Bewegung oder Haltung des Benutzers während der Benutzung des Trainingsgeräts zu erkennen und in diesem Fall dem Benutzer oder dem Trainingsgerät eine entsprechende Rückmeldung auszugeben. Das aus der Druckschrift bekannte Verfahren und das System zur Überwachung der Haltung eines Benutzers an einem Trainingsgerät ist auf die Erfassung der Bewegung und Haltung des Benutzers und/oder von ihm beaufschlagter Bauteile des Trainingsgeräts beschränkt und das Trainingsgerät selbst erlaubt keine aktive Anregung des Bewegungsapparat des Benutzers derart, dass eine Anregungsfunktion aufgeprägt wird.

Die US 2008/0161169 A1 beschreibt eine Vorrichtung zum computerunterstützten Training der die Wirbelsäule unterstützenden Muskulatur. Sie weist eine Basis auf, auf der mittels verschwenkbarer Ausleger eine Plattform angeordnet ist. Auf dieser ist eine um eine orthogonal zu der vorgenannten Plattform verlaufende Achse drehbare Grundplatte angeordnet, auf der der Benutzer der aus der vorgenannten Druckschrift bekannten Trainingsvorrichtung steht. Die Grundplatte ist um die vorgenannte Achse mittels einer Antriebseinrichtung computergesteuert drehbar. Auf der Grundplatte sind Fußhalterungen vorgesehen, welche die Füße des Benutzers auf der Grundplatte fixieren. An der Grundplatte ist eine Hüftabstützvorrichtung angeordnet, die aus einem vertikal zur Grundplatte verlaufenden Stützbalken und einem daran befestigten Hüftgurt besteht. Zum Training des Benutzers wird zuerst die Plattform, welche die drehangetriebene Grundplatte der Vorrichtung trägt, solange verschwenkt, bis die Plattform unter einem vorgegebenen Winkel zur Basis der Vorrichtung geneigt verläuft. Dann wird die Grundplatte um ihre dann geneigt zur Vertikalen verlaufende Drehachse gedreht. Hierdurch wird dann die das Rückgrat stabilisierende Muskulatur beaufschlagt, was in einer muskelverstärkenden Übung resultiert. Die Hüftabstützvorrichtung bewirkt dabei, dass der Benutzer auf der Grundplatte gerade steht und sein Körper bei dem Training nicht hin- und herschaukeln kann. Eine derartige Vorrichtung mag zwar zum Training der Rückmuskulatur eines Benutzers geeignet sein, sie ist aber nicht zum Training der Muskulatur der unteren Extremitäten des Benutzers, insbesondere der Fuß- und/oder Unterschenkelmuskulatur geeignet, da die Füße des Benutzers stationär auf der sich drehenden Grundplatte der Vorrichtung stehen, ohne dass auf sie ein dynamisches Drehmoment ausgeübt wird, da zwischen den in den Fußhalterungen der Grundplatte aufgenommenen Füßen des Benutzers und der durch die Antriebseinrichtung drehangetriebenen Grundplatte keine Relativbewegung gegeben ist.

Die US 2011/0256983 A1 beschreibt ein Rehabilitationssystem, welches ein Grundgestell mit einer Basisplattform aufweist, in die zwei Dreh-/Kippeinrichtungen eingelassen sind. Jede der beiden vorgenannten Einrichtun-gen weist eine in einem Rahmen kardanisch aufgehängte Trittplatte auf, die um eine erste, in Fußlängsrichtung verlaufende Achse und eine zweite, hierzu querverlaufende Achse beweglich sind. An jede der beiden Achsen greift jeweils ein Aktuator an, der es erlaubt, die um die jeweilige Achse erfolgende Bewegung der Trittplattform aktiv zu verstärken oder zu dämpfen. Auf jeder Trittplattform ist eine Fußhalterung angeordnet, mit der jeweils ein Fuß des Benutzers lagefixierbar ist. Bei einer entsprechenden Antriebsbewegung der Aktuatoren kann jeweils ein Drehmoment auf den Fuß des Benutzers aufgebracht werden, so dass sich der Knöchel des Fußes des Benutzers um eine oder beide Achsen dreht. Eine derartige Vorrichtung erlaubt zwar die Einleitung eines Drehmoments in den von ihr beaufschlagten Fuß des Benutzers. All den von der bekannten Vorrichtung ausgeübten Drehbewegungen des Fußes des Benutzers ist jedoch gemeinsam, dass sich der Fuß des Benutzers nur um einen zentralen Punkt, der durch den Kreuzungspunkt der in Fußlängsrichtung mit der in Fußquerrichtung verlaufenden Achse definiert ist, kippt. Dies ist ausreichend, um den von dem bekannten Trainer bewirkten Zweck, nämlich als ein virtueller Knöchel- und Gleichgewichtstrainer (Virtual Ankle and Balance Trainer (VABAT)) zu dienen, zu erfüllen: Die primäre Aufgabe des bekannten Trainers ist es nämlich, an neurologischen Störungen leidenden Patienten zu unterstützen, eine motorische Kontrolle des Knöchels wieder anzutrainieren oder komplexere Aufgaben wie ein Koordinationstraining, um eine reziproke motorische Kontrolle zu verbessern, die Fähigkeit, rasch ein Drehmoment in unterschiedliche Richtungen aufzubauen, Knöchel-Dorsiflexionen oder -Plantarflexionen in unterschiedlichen Graden von Fersen und Knieextensionen zu koordinieren, zu bewältigen. Hierzu soll die Bewegung des Fußes oder der Füße des Benutzers aktiv unterstützt werden. Die zweite Funktion eines derartigen Trainers besteht darin, Patienten, die an neurologischen Störungen leiden, zu unterstützen, ihren Gleichgewichtssinn durch wiederholte Übungen einer Verschiebung ihres Druckschwerpunkts zu trainieren.

Die bekannten Vorrichtungen erlauben zwar ein Training, insbesondere der Beinund Rückenmuskulatur eines Benutzers dieser Vorrichtungen, indem die entsprechenden Muskeln des Bewegungsapparats durch die von der jeweiligen Vorrichtung erzeugten Schwingungen und/oder Vibrationen beaufschlagt werden. Es ist aber nicht oder nur schwer möglich, auf den Benutzer der Vorrichtung ein definiertes Drehmoment zu übertragen, welches vom Benutzer durch entsprechende Muskelaktionen kompensiert werden kann. Die bekannten Vorrichtungen besitzen insbesondere den Nachteil, dass sie es nicht oder nur in einem geringen Umfang erlauben, insbesondere auf die unteren Extremitäten eines auf dem Grundelement stehenden Benutzers ein hinreichend großes Drehmoment aufzubringen. Dies gilt beispielsweise insbesondere für die Heber-Muskulatur des Unterschenkels. Steht der Benutzer mit seinen Füßen auf der Vorrichtung, so ist das maximal nutzbare Drehmoment durch den Abstand des Fersenendes zu dem als Drehpunkt fungierenden Sprunggelenk (Heber- Muskulatur) bzw. den Abstand der Zehenspitzen zum Sprunggelenk (Beuger-Muskulatur), multipliziert mit der Körpermasse des Benutzers, gegeben. Eine derartige Limitierung ist insbesondere beim Training der Heber-Muskulatur von Nachteil, da der Abstand zwischen Ferse und Sprunggelenk klein ist. Insbesondere bei neurologischen Erkrankungen, Hemiparese/Parese und bei Spastiken ist aber das Training dieser Heber-Muskulatur von großer Bedeutung, da hierbei eine große Asymmetrie zwischen dem Agonisten, der Wadenmuskulatur, und dem Antagonisten, dem Tibialis Anterior, gegeben ist. Ist die sprunggelenksstabilisierende Muskulatur, wie beispielsweise in den oben genannten Beispielen schlecht und/oder sehr einseitig ausgebildet, so können auch auf die folgenden Gelenke (Knie, Hüfte, Rücken) keine ausreichend (normal) großen Drehmomente übertragen bzw. internen Muskelkräfte erzeugt werden. Somit ist die Muskulatur des Oberschenkels, der Hüfte und des Rumpfes ebenfalls schlecht und/oder sehr Einseitig ausgebildet. Um ausreichend große Momente und damit muskulären Kräfte in diesen Körperregionen zu erzeugen muss daher das Sprunggelenk zur Momentenübertragung unterstützt bzw. stabilisiert werden.

Die FR 2 809 615 A1 beschreibt eine Vorrichtung zum Training des Bewegungsapparates eines Benutzers, welche einen auf Schienen gelagerten Schlitten aufweist, der von einer Antriebseinrichtung entlang dieser Führungsschienen hinund herbewegt werden kann. Auf dem Schlitten sind zwei Fußhalter angeordnet, die um ein Gelenk drehbar mit dem Schlitten verbunden sind. Eine Anhebung und/oder Absenkung des oder der auf dem Schlitten aufsetzenden Füße in seiner oder ihrer Gesamtheit findet somit nicht statt.

Dies gilt auch für die aus der JP 2004 261 256 A bekannten Vorrichtung. Bei dieser ist ein Grundelement vorgesehen, welches von einem Exzenterantrieb derart antreibbar ist, dass das Grundelement über eine vertikale Trageachse verschwenkbar ist, an deren oberen Ende ein das Grundelement tragendes Gelenk angeordnet ist. Der Fuß des Benutzers ist mit einem Riemen mit dem Grundelement verbunden.

Die US 2005/251067 A1 beschreibt eine Bewegungsvorrichtung, welche dazu dient, den venösen Blutfluss in den unteren Extremitäten eines Benutzers zu verbessern, um hierdurch eine bestimmte Art einer Thrombose ("Deep Vain Thrombosis" [DVT]) oder einen pulmonären Embolismus, ein Ödem der unteren Extremitäten oder andere damit einhergehende und verbundene Zustände zu verhindern. Hierzu weist die Vorrichtung eine Liegefläche auf, auf der der Benutzer waagrecht liegt. Seine Fußsohlen weisen somit im Ruhezustand vertikal nach oben, während sein Unterschenkel horizontal liegt. An der Grundfläche ist ein Grundelement vorgesehen, welches über ein Gelenk beweglich verbunden wird. Dieses Grundelement wird von einem Exzenterantrieb beaufschlagt, so dass das Grundelement eine Schwingung durchführt. Die Füße des Benutzers sind über eine Fußschlaufe mit dem Grundelement verbunden, so dass hier eine Drehung des Fußes um das Fersengelenk erzwungen wird.

Die US 5 176 598 A beschreibt eine Vorrichtung, die zwei Fußhalterungen aufweist, die parallel zueinander in einem Gehäuse der Vorrichtung angeordnet sind. Ein Antriebsmotor überträgt eine Vibrationsbewegung über ein Getriebe auf die Fußhalterungen. Diese sind auf einer Achse gelagert, so dass bei einer durch den Motor bewirkten Auf- und Abbewegung des vorderen Teils der Fußhalterungen diese eine Schwenkbewegung um diese Achse durchführen. Auch bei dieser Vorrichtung werden daher der oder die Füße des Benutzers nicht in ihrer Gesamtheit angehoben oder abgesenkt. Die zu den vorstehenden Druckschriften gemachten Ausführungen gelten hier entsprechend.

Es ist daher Aufgabe der vorliegenden Erfindung, eine Vorrichtung der eingangs genannten Art derart weiterzubilden, dass in einfacher Art und Weise die Erzeugung und/oder Übertragung eines verbesserten Drehmoments auf die Beinund/oder Fußmuskulatur des Benutzers, insbesondere der Heber-Muskulatur, ermöglicht wird.

Diese Aufgabe wird erfindungsgemäß dadurch gelöst, dass die Vorrichtung eine Fixationseinrichtung mit mindestens einem Fußhalter aufweist, durch welche der oder beide Füße des Benutzers auf dem Grundelement lagefixierbar ist oder sind, und dass der Fusshalter der Fixationseinrichtung derart konfiguriert ist, das der Benutzer während der periodischen Auf- und Abbewegung der Grundplatte der Vorrichtung derart seine Lage verändern kann, dass eine Projektion seines Körperschwerpunktes außerhalb der Unterstützungsfläche des oder der Füße liegt.

Durch die erfindungsgemäßen Maßnahmen wird in vorteilhafter Art und Weise eine Vorrichtung geschaffen, welche es erlaubt, auf die unteren Extremitäten des Benutzers, insbesondere auf dessen Fuß- und/oder Unterschenkelmuskulatur, ein für ein Training und/oder eine Analyse des Bewegungsapparats hinreichend großes Drehmoment aufzubringen. Indem nun erfindungsgemäß vorgesehen ist, dass der auf dem Grundelement der erfindungsgemäßen Vorrichtung aufsetzende Fuß und/oder der Unterschenkel des Benutzers durch die Fixationseinrichtung Fixiereinrichtung lagefixiert ist, wird eine hinreichend gute Übertragung des von der Vorrichtung erzeugten Drehmoments auf den Benutzer gewährleistet. Durch die periodische Bewegung des Grundelements der erfindungsgemäßen Vorrichtung, welche derartig ist, dass hier bei der Fuß oder beide auf dem Grundelement aufsetzenden Füße des Benutzers durch die Bewegung des Grundelements periodisch in ihrer Gesamtheit angehoben und abgesenkt werden, wird eine hinreichend große Bewegungsamplitude des oder der Füße des Benutzers und somit auch von dessen Unterschenkeln erreicht, was in einem effektiven Training des Bewegungsappartes des Benutzer und/oder einer aussagekräftigen Analyse desselben resultiert. Die erfindungsgemäße Vorrichtung erlaubt in vorteilhafter Art und Weise eine Trainings- und/oder Analysefrequenz zwischen 0,1 und 50 Hz, vorzugsweise zwischen 3 und 35 Hz, so dass eine Vielzahl von Wiederholungen innerhalb von kurzer Zeit gewährleistet ist.

Ein weiterer Vorteil der erfindungsgemäßen Vorrichtung besteht darin, dass - da der oder die Füße des Benutzers durch die erfindungsgemäß vorgesehene mindestens eine Fixiereinrichtung fixiert sind - sich der Benutzer deutlich weiter nach vorne und hinten beugen kann, als es ihm ohne eine derartige Fixation möglich wäre. Die erfindungsgemäße Fixiereinrichtung ermöglicht es, dass die Projektion seines Schwerpunktes außerhalb der Unterstützungsfläche des oder der Füße liegen kann. Somit können durch die Fixation des oder der Füße auf dem sich auf- und abwärtsbewegenden Grundelement Drehmomente und daraus resultierende muskuläre Kräfte erzeugt werden, die deutlich größer sind, als sie bei einem Training ohne die erfindungsgemäße Vorrichtung möglich wären. Diese deutlich größeren Drehmomente werden dann insbesondere auch an die nachfolgenden Gelenke des Bewegungsapparates übertragen. In Kombination mit diesen großen Drehmomenten und den daraus resultierenden muskulären Kräften ist somit ein effizientes Training nicht nur der Muskulatur des Fußes bzw. des Unterschenkels, sondern auch der nachfolgenden Muskelgruppen möglich. Die erfindungsgemäße Vorrichtung eignet sich daher insbesondere zum Training der Heber-Muskulatur.

Durch die erfindungsgemäße Fixierung des Fußes auf der erfindungsgemäßen Vorrichtung kann also nicht nur im Sprunggelenk ein größeres Drehmoment erzeugt werden, sondern auch in allen nachfolgenden Gelenken (Knie, Hüfte, Rumpf). Die erfindungsgemäße Vorrichtung kann somit zum Training des gesamten Bewegungsapparates genutzt werden.

Durch die erfindungsgemäßen Maßnahmen wird des weiteren in vorteilhafter Art und Weise eine Vorrichtung geschaffen, die mithilfe der erzeugten Drehmomente entsprechende muskuläre Kräfte aktiviert. Sie erlaubt in Verbindung mit den von ihr erzeugten Schwingungen und/oder Vibrationen ein Vibrationstraining und ermöglicht es, Muskelgruppen zu trainieren, die ohne Einsatz der erfindungsgemäßen Maßnahmen nicht oder nur unzureichend erreicht werden können.

Eine vorteilhafte Weiterbildung der Erfindung sieht vor, dass die Fixationseinrichtung auf dem Grundelement der Vorrichtung angeordnet ist. Eine derartige Maßnahme besitzt den Vorteil, dass hierdurch die Fixationseinrichtung der Bewegung des Grundelements folgt, so dass während jeder Phase des Trainings und/oder der Analyse des Bewegungsapparats eines Benutzers eine hinreichende Fixation zumindest seiner Füße gegeben ist.

Eine weitere vorteilhafte Weiterbildung der Erfindung sieht vor, dass die Fixationseinrichtung grundelementextern, insbesondere auf einem Sockel der Vorrichtung, angeordnet ist. Eine derartige Maßnahme besitzt den Vorteil, dass die direkt angetriebene bewegte Masse durch diese zusätzliche Fixationseinrichtung nicht erhöht wird und somit nicht kompensiert werden muss. Zudem kann das Gerät, auch ohne die zusätzliche Fixation zu nutzen, benutzt werden.

Eine weitere vorteilhafte Weiterbildung der Erfindung sieht vor, dass die Vorrichtung mindestens einen Sensor zur Ermittlung einer Antwortfunktion eines Benutzers der Vorrichtung auf die durch das Grundelement auf ihn aufgeprägte Anregungsfunktion, dessen oder deren Sensorsignale einer Auswerteeinrichtung der Vorrichtung zugeführt sind, aufweist. Mit Hilfe des oder der Sensoren können in vorteilhafter Art und Weise die resultierenden Momente gemessen oder berechnet werden. Auf diese Weise kann eine Dokumentation des Trainings, des Trainingserfolges und/oder eine Trainingssteuerung erfolgen. Zudem kann das momentan erzeugte Ergebnis dem Trainierenden zur Verfügung gestellt werden, beispielsweis mittels Display, LED-Anzeige oder auch akustisch oder haptisch, damit dieser sein momentanes Training optimieret (Rückkopplungs- bzw. Bio-Feedback System).

Eine weitere vorteilhafte Weiterbildung der Erfindung sieht vor, dass die Vorrichtung mindestens einen weiteren Sensor aufweist, durch den die Position und/oder der Bewegungszustand des die Anregungsfunktion der Vorrichtung auf den Benutzer aufprägenden Grundelements erfassbar ist und dessen oder deren Sensorsignale der Auswerteeinrichtung zuführbar ist, und dass durch die Auswerteeinrichtung ein Vergleich der durch das Grundelement auf den Benutzer aufgeprägten Anregungsfunktion und der Antwortfunktion des Benutzers durchführbar ist, indem die Auswerteeinrichtung die Amplitude und/oder die Phasenlage dieser beiden Funktionen zur Bestimmung von Parametern des Bewegungsapparates des Benutzers analysiert.

Durch die erfindungsgemäßen Maßnahmen wird in vorteilhafter Art und Weise erreicht, dass die Anregungsfunktion, welche die erfindungsgemäße Vorrichtung auf ihren Benutzer ausübt, und dessen Antwortfunktion darauf zu einander in Beziehung gesetzt werden können. Dadurch kann eine Übertragungsfunktion, welche die vom Grundelement auf den Benutzer aufgeprägte Anregungsfunktion auf die Antwortfunktion des Benutzers abbildet, bestimmt werden. Aus dieser Übertragungsfunktion lassen sich dann Rückschlüsse über die Funktion - und insbesondere über Fehlfunktionen - des Bewegungsapparates eines bestimmten Benutzers ziehen und in einfacher Art und Weise viele Funktionen diagnostizieren. Ausgehend davon kann dann das Training des Bewegungsapparates des Benutzers verbessert werden.

Weitere vorteilhafte Weiterbildungen der Erfindung sind Gegenstand der Unteransprüche.

Weitere Einzelheiten und Vorteile der Erfindung sind den Ausführungsbeispielen zu entnehmen, die im Folgenden anhand der Figuren beschrieben werden.

Es zeigen:
- Figur 1:: ein erstes Ausführungsbeispiel einer Vorrichtung,
- Figur 2:: eine Draufsicht auf das erste Ausführungsbeispiel aus der Richtung II der Figur 1,
- Figuren 3a und 3b:: eine schematische Ansicht der Figuren 1 und 2 zusammen mit einer Darstellung der Beine des Benutzers,
- Figuren 4a bis 4c:: eine schematische Darstellung der Funktionsweise des ersten Ausführungsbeispiels,
- Figur 5:: eine zweite Ausführungsform einer Fixationseinrichtung,
- Figur 6:: eine dritte Ausführungsform einer Fixationseinrichtung,
- Figur 7:: eine vierte Ausführungsform einer Fixationseinrichtung,
- Figuren 8a-8c:: eine fünfte Ausführungsform einer Fixationseinrichtung in drei verschiedenen Variationen,
- Figuren 9a-9h:: eine sechste Ausführungsform einer Fixationseinrichtung,
- Figuren 10a, 10b:: eine siebte Ausführungsform einer Fixationseinrichtung,
- Figuren 11a-11c:: eine achte Ausführungsform einer Fixationseinrichtung,
- Figuren 12a-12c:: eine neunte Ausführungsform der Fixationseinrichtung,
- Figur 13:: ein zweites Ausführungsbeispiel einer Vorrichtung,
- Figur 14:: ein drittes Ausführungsbeispiel einer Vorrichtung,
- Figur 15:: eine Draufsicht auf das dritte Ausführungsbeispiel,
- Figuren 16a-16b:: eine schematische Darstellung der Benutzungsweise des dritten Ausführungsbeispiels,
- Figur 17:: ein viertes Ausführungsbeispiel der Vorrichtung,
- Figur 18:: eine Draufsicht auf das vierte Ausführungsbeispiel der Vorrichtung,
- Figuren 19a-19c:: ein fünftes Ausführungsbeispiel der Vorrichtung, welches nicht Gegenstand der beanspruchten Erfindung ist,
- Figuren 20a-20c:: ein sechstes Ausführungsbeispiel der Vorrichtung, welches nicht Gegenstand der beanspruchten Erfindung ist,
- Figuren 21a-21c:: ein siebtes Ausführungsbeispiel der Vorrichtung, welches nicht Gegenstand der beanspruchten Erfindung ist,
- Figuren 22a-22c:: ein achtes Ausführungsbeispiel der Vorrichtung, welches nicht Gegenstand der beanspruchten Erfindung ist,
- Figur 23:: ein neuntes Ausführungsbeispiel der Vorrichtung,
- Figur 24;: eine Draufsicht auf das neunte Ausführungsbeispiel aus der Richtung XXIV der Figur 1, und
- Figuren 25a-25c:: ein zehntes Ausführungsbeispiel der Vorrichtung.

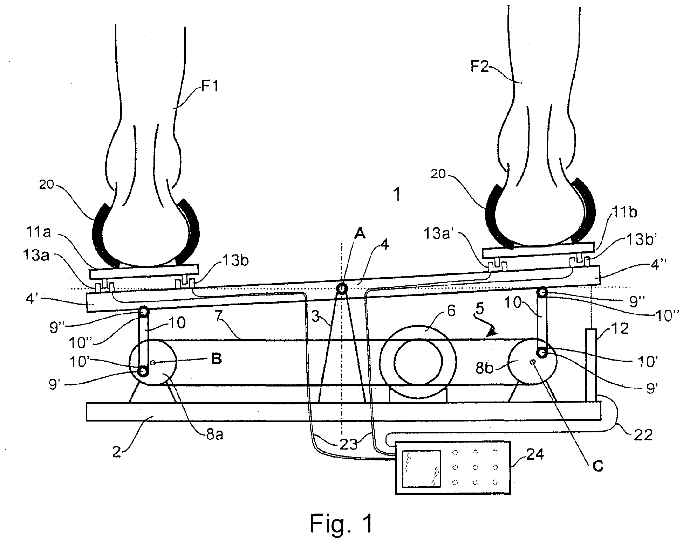

Die Figuren 1 bis 4c zeigen nun ein erstes Ausführungsbeispiel einer allgemein mit 1 bezeichneten Vorrichtung für ein Training und/oder eine Analyse des Bewegungsapparats eines Lebewesens, insbesondere eines Menschen. Die Vorrichtung 1 besitzt einen Sockel 2 mit einem Ausleger 3, an dem ein um eine Achse A drehbar gelagertes Grundelement 4 angeordnet ist, welches hier als Grundplatte ausgeführt ist. Das im gezeigten Ausführungsbeispiel als Wippe fungierende Grundelement 4 wird von einer Antriebseinrichtung 5 angetrieben, welche dazu dient, das Grundelement 4 in periodische und/oder aperiodische Schwingungen und/oder Vibrationen zu versetzen. Im hier gezeigten Fall weist die Antriebseinrichtung 5 einen Elektromotor 6 auf, welcher über einen Riemen 7 zwei um Achsen B und C drehbar gelagerte Antriebsscheiben 8a und 8b antreibt. An einem ersten Anlenkpunkt 9' ist jeweils ein erstes Ende 10' eines Pleuels 10 angelenkt. Ein zweites Ende 10" eines jeden Pleuels 10 greift über einen weiteren Anlenkpunkt 9" an der Grundplatte 4 an, so dass durch eine Drehbewegung der Antriebsscheiben 8a, 8b die Grundplatte 4 in sinusförmige Schwingungen versetzt wird.

Dem Fachmann ist aus der obigen Beschreibung ebenfalls klar ersichtlich, dass die hier beschriebene Lagerung des Grundelements 4 sowie die Ausbildung der Antriebseinrichtung 5 nur exemplarischen Charakter besitzen. So ist es zum Beispiel auch möglich, dass das Grundelement 4 über entsprechende Führungen (nicht gezeigt) auf- und abbeweglich gelagert ist. Auch ist es nicht erforderlich, dass das Grundelement 4 an zwei Stellen angetrieben wird. Ein Antrieb des Grundelements 4 an nur einer Stelle oder an mehr als zwei Stellen ist ebenfalls möglich, genauso wie ein Antrieb desselben über mehr als eine Antriebseinrichtung. Ebenso wenig ist es zwingend, dass das Grundelement 4 mit einer periodischen Anregung beaufschlagt wird. Auch eine aperiodische Beaufschlagung des Grundelements 4, insbesondere eine stochastische oder impulsförmige Beaufschlagung, welche nicht Gegenstand **der beanspruchten Erfindung ist, sofern sie nur einmalig erfolgt,** ist möglich. Ebenso ist es denkbar, dass das Grundelement 4 anstelle von sinusförmigen Schwingungen oder zusätzlich dazu mit Vibrationen beaufschlagt wird. Vorzugsweise wird hierbei eine Frequenz verwendet, die im Bereich von 0 bis 50 Hz bzw. 10 bis 35 Hz liegt. Eine Anregung im erstgenannten Frequenzbereich besitzt den Vorteil, dass hierdurch der Regelkreis des postularen Systems gezielt untersucht werden kann. Der zweitgenannte Bereich erlaubt eine effiziente Analyse der Steifigkeit und Dämpfung des Bewegungsapparates. Dem Fachmann ist klar ersichtlich, welche Modifikationen er an dem beschriebenen

Aufbau vorzunehmen hat, um bei der beschriebenen Vorrichtung 1 eine oder mehrere der vorstehend beschriebenen Möglichkeiten vorzusehen. Im folgenden soll jedoch der Einfachheit halber weiterhin von dem in Figur 1 gezeigten und vorstehend beschriebenen Aufbau ausgegangen werden, da dies die Allgemeingültigkeit der nachfolgenden Ausführungen nicht beeinträchtigt.

Die Antriebsbewegung des Elektromotors 6 sowie deren Übertragung über die Antriebsscheiben 8a, 8b und die Pleuel 10 auf das Grundelement 4 bewirkt, dass diese eine sinusförmige Bewegung durchführt, wobei einander gegenüberliegende Enden 4' und 4" des Grundelements 4 eine Phasenverschiebung von 180 ° aufweisen. Der Bewegungsapparat eines auf dem Grundelement 4 stehenden Benutzers wird somit mit einer sinusförmigen Anregung beaufschlagt, woraufhin dieser nun versucht, die Anregungsbewegungen dem Grundelement 4 durch entsprechende Reaktionen seines Bewegungsapparates zu kompensieren. Vorzugsweise ist vorgesehen, dass diese sinusförmige Anregungsfunktion eine Frequenz im Bereich von 0,01 bis 50 Hz besitzt, wobei der bevorzugte Frequenzbereich zwischen 3 und 35 Hz liegt. Vorzugsweise beträgt die Amplitude zwischen 0,1 und 10 mm.

Auf dem Sockel 2 der Vorrichtung 1 ist ein Abstandssensor 12 angeordnet, durch den die Auslenkung des Grundelements 4 erfassbar ist. Sein Sensorsignal wird über eine Signalleitung 22 zu einer Auswerteeinrichtung 24 geführt und dort entsprechend verarbeitet. Der Abstandssensor 12 erlaubt eine kontinuierliche, quasi-kontinuierliche oder zu diskreten Zeitpunkten stattfindende Erfassung der Lage des Grundelements 4, wobei aus dem zeitlichen Verlauf der Auslenkungsamplituden des Grundelements 4 die Auswerteeinrichtung deren Frequenz und/oder Amplitude, insbesondere die zur aktuellen Auslenkung des Grundelements korrelierte Momentanamplitude, sowie daraus ableitbare Bewegungsparameter des Grundelements 4 ermitteln werden können. Bevorzugt wird hierbei, dass die Amplitude und/oder die Frequenz der Auslenkung des Grundelements 4 in seiner Wirkrichtung, im hier gezeigten Fall also in der horizontalen Richtung, erfasst bzw. ermittelt werden.

Anstelle des Abstandssensors 12 kann auch ein anderer Sensortyp, zum Beispiel ein Kraft-, Druck-, Weg-, Geschwindigkeits- oder Beschleunigungssensor eingesetzt werden, solange gewährleistet ist, dass durch den entsprechenden Sensor die Anregungsbewegung des Grundelements 4 der Vorrichtung 1 in seiner Wirkrichtung erfassbar ist.

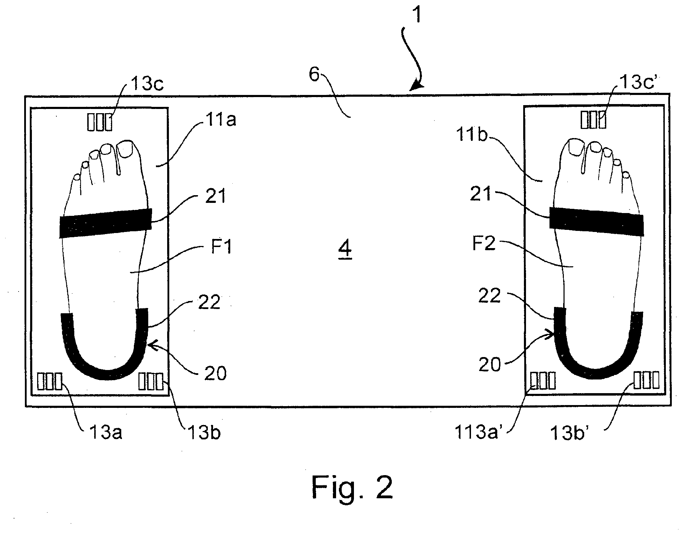

Wie aus den Figuren 1 und 2 ersichtlich, weist das Grundelement 4 zwei Trittplattformen 11a, 11b auf, welche dazu dienen, eine definierte Position des Benutzers auf dem als Grundplatte ausgebildeten Grundelement 4 festzulegen. Selbstverständlich sind diese Trittplattformen 11a, 11b nicht zwingend erforderlich. Es ist durchaus möglich, dass der Benutzer der Vorrichtung 1 direkt auf der Grundplatte steht.

Eine derartige Vorrichtung ist aus der EP 2 160 168 B1 der Anmelderin bekannt, auf die zur Vermeidung von Wiederholungen bezug genommen und deren Offenbarung durch diese Bezugnahme zum Gegenstand dieser Anmeldung gemacht wird. Dem Fachmann ist aus obiger Beschreibung ersichtlich, dass der oben beschriebene Aufbau in weiten Grenzen variiert werden kann. Es ist auch möglich, den in der EP 0 929 284 bekannten Aufbau einer Vorrichtung zur Erzeugung von periodischen und/oder aperiodischen Anregungen, insbesondere von periodischen und/oder aperiodischen Schwingungen und/oder Vibrationen zu verwenden.

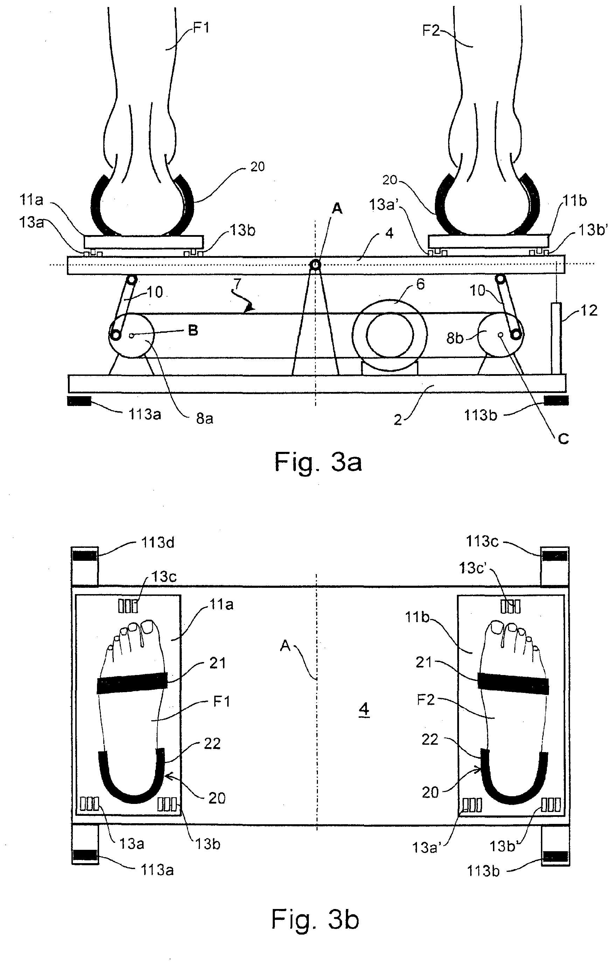

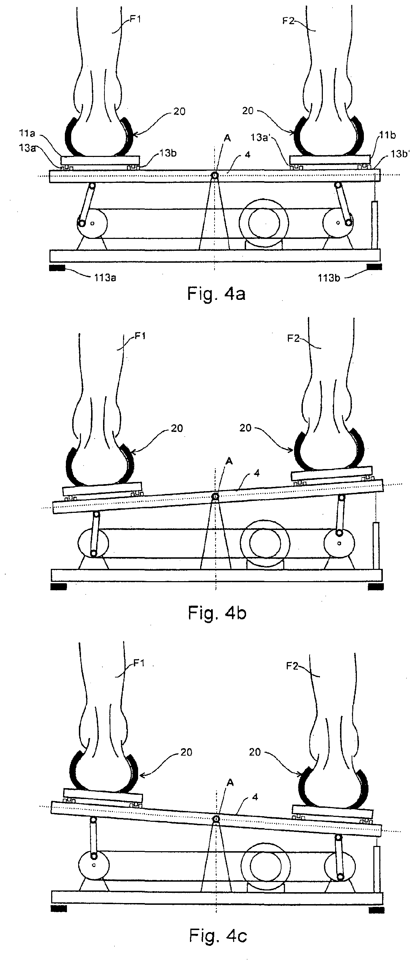

Um nun ein Drehmoment zu erzeugen und auf den Benutzer der beschriebenen Vorrichtung 1 zu übertragen, ist vorgesehen, dass der Benutzer nicht - wie bei der bekannten Vorrichtung - einfach auf dem Grundelement 4 bzw. auf den Trittplattformen 11a, 11b steht, sondern dass seine Füße F1, F2 mittels jeweils einer Fixationseinrichtung 20 darauf fixiert sind, so dass einem Abheben der Fußsohlen von dem oszillierenden und/oder vibrierenden Grundelement 4 bzw. den mit dieser verbundenen Trittplattformen 11a, 11b wenn nicht verhindert, dann doch zumindest in hohem Umfang entgegengewirkt wird. Im einfachsten Fall wird dies dadurch erreicht, dass - wie am besten aus Figuren 3a, 3b und 4a-4c ersichtlich - an den Trittplattformen 11a, 11b jeweils eine den entsprechenden Fuß F1 bzw. F2 fest umschließende Schlaufe 21 angeordnet ist, welche die Füße F1 bzw. F2 auf der Trittplattform 11a bzw.11b fixiert. Eine derartige Schlaufe 21 ist für einfache Anwendungsfälle durchaus ausreichend. Es wird jedoch bevorzugt, dass die Fixierung der Füße F1, F2 auf den Trittplattformen 11a, 11b nicht nur durch die Schlaufe 21 alleine, sondern durch die Fersen der Füße F1, F2 umschließende Fersenhalter 22 erfolgt. Die Schlaufe 21 und der Fersenhalter 22 bilden somit Elemente der Fixationseinrichtung 20 aus, welche den jeweiligen Fuß F1 bzw. F2 umfasst und diesen somit gegen ein unerwünschtes Abheben gesichert auf den jeweiligen Trittplattformen 11a bzw. 11b fixiert.

Bewegt sich nun das als Wippe ausgestaltete Grundelement 4 um seine Drehachse A, so bewegt sich der in den Figuren 3a und 3b rechte Teil des Grundelements 4 und damit die mit ihm verbundene Trittplattform 11a nach unten, während sich gleichzeitig die linke Hälfte und somit die Trittplattform 11b nach oben bewegt. Die dabei durchlaufende Bewegungssequenz wird anhand der Figuren 4a bis 4c erläutert:

Die Figur 4a zeigt ein das Grundelement 4 am Ende seiner vorher beschriebenen Bewegung, das heißt, das Grundelement 4 verläuft in Figur 4a von links oben nach rechts unten. Die Figur 4b zeigt das Grundelement 4 in seiner Ausgangslage, das heißt, in seiner Waagrecht-Stellung, während die Figur 4c die Stellung des Grundelements 4 am Ende der darauffolgenden Wippbewegung zeigt, in der das Grundelement 4 von links unten nach rechts oben verläuft. Wie man aus einem Vergleich der Figuren 4a und 4b erkennt, bewirkt die Fixationseinrichtung 20, dass auf die Füße F1, F2 ein im Uhrzeigersinn wirkendes Drehmoment ausgeübt wird, welches bewirkt, dass diese um eine in Fußlängsrichtung verlaufende Achse um das Sprunggelenk gedreht werden. Das vegetative Nervensystem des Benutzers reagiert auf eine Verdrehung der Füße F1, F2 und regt die entsprechenden Muskeln zu einer Kompensationsbewegung an, wodurch deren Training erreicht wird.

Nachdem das Grundelement 4 am unteren Totpunkt der vorstehend beschriebenen und in Figur 4a dargestellten Wippbewegung - an diesem Punkt ist das vorgenannte, rechtsgerichtete Drehmoment am größten - seine Bewegungsrichtung umgekehrt und sich seine rechte Hälfte nun nach oben und folglich seine linke Hälfte nach unten bewegt, gelangt das Grundelement 4 nach Durchlaufen seiner in Figur 4b gezeigten Ruhestellung - in dieser wird kein Drehmoment von der Vorrichtung 1 auf die Füße F1, F2 übertragen - in die in Figur 4c gezeigte Position, in der auf die beiden Füße F1, F2 ein entgegengesetzt orientiertes, also linksdrehendes Drehmoment ausgeübt wird.

Zudem kann der Benutzer seinen Körperschwerpunk nach vorne oder nach hinten oder seitwärts verlagern. Da die Füße F1, F2 fixiert sind kann sich der Benutzer deutlich weiter nach vorne und hinten beugen als es ihm ohne eine derartige Fixation möglich wäre, da die Projektion seines Schwerpunktes nun außerhalb der Unterstützungsfläche des oder der Füße F1, F2 liegen kann. Somit können durch die Fixation der Füße F1, F2 Drehmomente und daraus resultierenden muskulären Kräfte erzeugt werden, die deutlich größer sind als sie bei einem Training ohne die Vorrichtung 1 möglich wären. Diese deutlich größeren Drehmomente werden dann insbesondere auch an die nachfolgenden Gelenke des Bewegungsapparates (Knie, Hüfte, Torso) übertragen. In Kombination mit diesen großen Drehmomenten und den daraus resultierenden muskulären Kräften und der überlagerten Schwingung ist somit ein effizientes Training nicht nur der Muskulatur des Fußes bzw. des Unterschenkels sondern auch der nachfolgenden Muskelgruppen möglich.

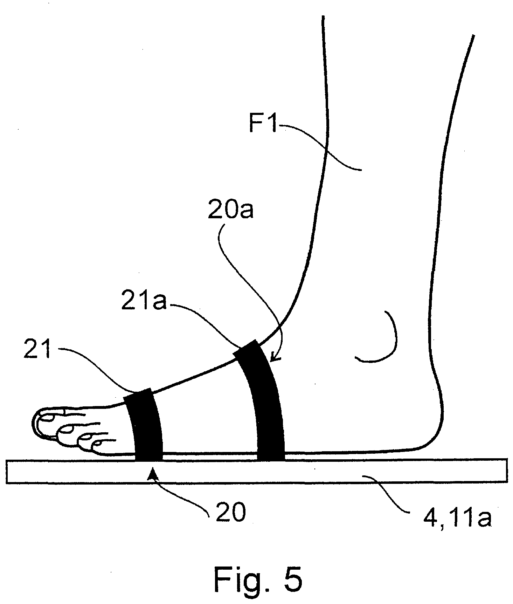

In den weiteren Figuren sind nun weitere Ausführungsformen der Fixationseinrichtung 20 dargestellt. Die Figur 5 zeigt eine zweite Ausführungsform der Fixationseinrichtung 20, welche nunmehr zwei mit den entsprechenden Trittplattformen 11a, 11b verbundene Schlaufen 21, 21a besitzt. Es ist offensichtlich, dass die Verwendung von nur einer oder zwei Schlaufen 21, 21a nicht zwingend ist. Vielmehr ist auch die Verwendung von drei oder mehr Schlaufen denkbar. Es ist ebenfalls auch offensichtlich, dass die Schlaufen 21, 21a in unterschiedlichen Breiten ausgeführt werden können und somit eine Schlaufe beispielsweise den gesamten oder einen Teil des Vorderfußes bedeckt. Die Schlaufe 21 der ersten Ausführungsform sowie die Schlaufen 21, 21a der zweiten Ausführungsform bilden somit eine Fußhalterung 20a der Fixationseinrichtung 20 aus.

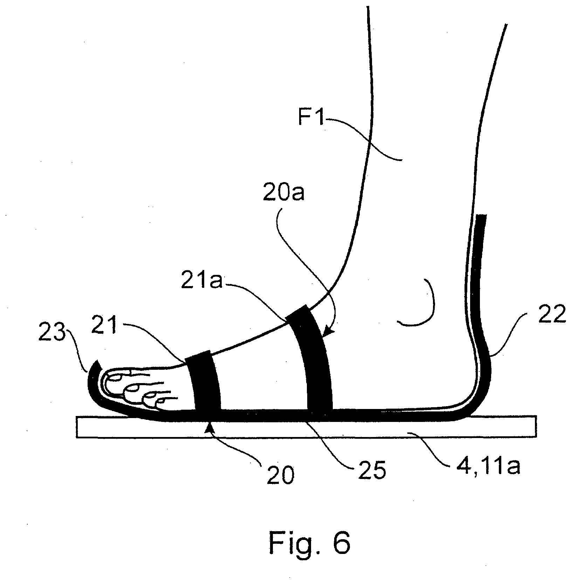

Die Figur 6 zeigt eine dritte Ausführungsform der Fixationseinrichtung 20. Diese weist eine sandalenartig ausgestaltete Fußhalterung 20a mit zwei Schlaufen 21, 21a sowie einen den Fersenbereich umschließenden sowie einen Teil des Unterschenkels abstützenden Fersenhalter 22 auf. An ihrem vorderen Ende weist die Fixationseinrichtung 20 einen den Zehenbereich umgebende Zehenhalter 23 auf. Außerdem ist ein Fußbett 25 vorgesehen, an dem die Schlaufen 21, 21a befestigt sind. Von Vorteil ist, wenn diese Fixationseinrichtung 20 - sowie auch alle anderen hier beschriebenen Fixationseinrichtungen 20 - vom den Grundelement 4 bzw. von den Trittplattformen 11a, 11b abnehmbar ausgestaltet ist. Hierzu weist sie optional nicht gezeigte Befestigungselemente auf, mit denen die Fixationseinrichtungen 20 an dem Grundelement 4 bzw. an der entsprechenden Trittplattform 11a, 11b befestigbar sind. Eine derartige einen Austausch ermöglichende Ausgestaltung besitzt den Vorteil, dass hierdurch in einfacher Art und Weise die Fixationseinrichtung 20 der Fußgröße des jeweiligen Benutzers angepasst werden kann.

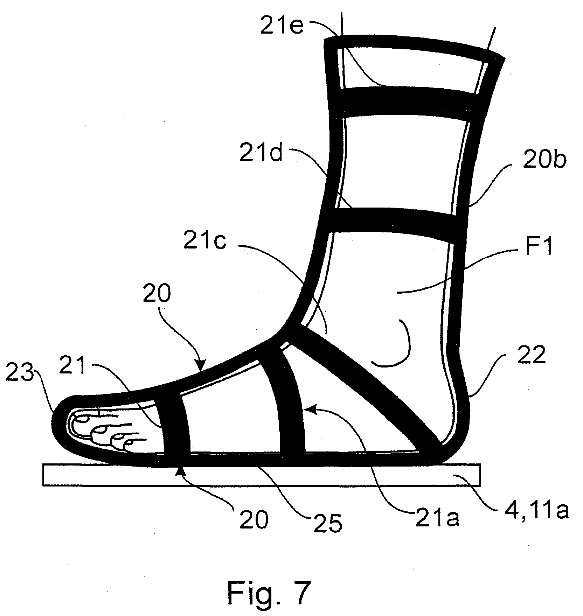

In Figur 7 ist eine vierte Ausführungsform der Fixationseinrichtung 20 dargestellt, ihrem Grundaufbau nach der dritten Ausführungsform der Figur 6 entspricht. Der wesentliche Unterschied zwischen der dritten und der vierten Ausführungsform ist, dass der Fersenhalter 22 höher gezogen ist als bei der dritten Ausführungsform, so dass dieser nicht nur den Bereich des Sprunggelenks, sondern auch einen Teil des Unterschenkels abstützt. Der in das Fußbett 25 übergehende Fersenhalter 23 ist mit weiteren Schlaufen 21d, 21e am Unterschenkel befestigt. Der obere Teil des Fersenhalters 22 bildet somit zusammen mit den Schlaufen 21d, 21e ein Unterschenkelhalter 20b der Fixationseinrichtung 20 aus. Eine weitere mit dem Fußbett 25 verbundene Schlaufe 21c fixiert den Fuß im Bereich seines Rists.

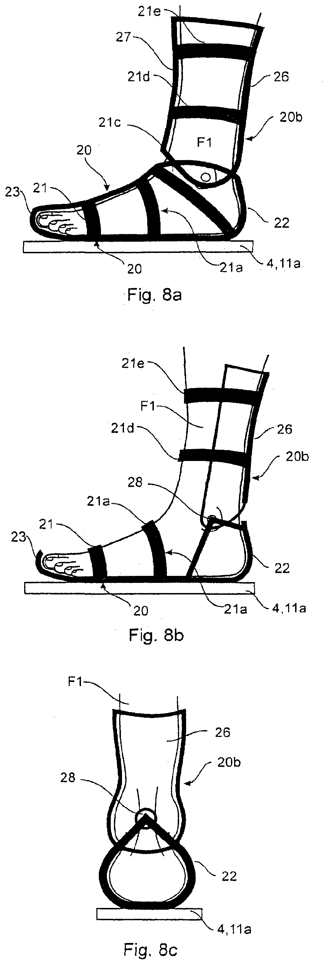

Die Figuren 8a bis 8c zeigen eine fünfte Ausführungsform einer Fixationseinrichtung 20 in drei unterschiedlichen Varianten, deren Grundaufbau demjenigen der vierten Ausführungsform der Figur 7 entspricht. Der wesentliche Unterschied zwischen diesen beiden Ausführungsformen ist, dass den den Unterschenkel umschließende Unterschenkelhalter 20b der Fixationseinrichtung 20 nicht nur - wie bei der Figur 7 - als Stütze ausgestaltet ist, sondern stiefelschaftartig mit einer rückwärtigen Stütze 26 und einer vorderen Lasche 27 (Figur 8a) oder als Halbschale (Figur 8b) ausgestaltet ist, welche durch die Streifen 21d und 21e zu dem stiefelartigen Schaft geschlossen werden können. Die - nicht zwingend erforderliche - Lasche 27 bewirkt eine Abstützung des Unterschenkels nach vorne.

Ein weiterer Unterschied ist, dass der Fußhalter 20a der Fixationseinrichtung 20 mit dem Unterschenkelhalter 20b über eine Gelenkanordnung 28 verbunden ist. Hierdurch wird ermöglicht, dass der Unterschenkel um das Sprunggelenk relativ zu dem lagefixierten Fuß F1, F2 beweglich ist, wodurch ein weiterer Freiheitsgrad beim Training der entsprechenden Muskeln eröffnet wird. Die Gelenkanordnung 28 kann derart ausgebildet sein, dass der maximale Neigungswinkel des Unterschenkelhalters 20b zum Fußhalter 20a beschränkt oder beschränkbar ist. Außerdem kann vorgesehen sein, dass - wie in Figur 8c dargestellt - die Gelenkanordnung 28 derart ausgebildet ist, dass der Unterschenkelhalter 20b nicht nur in einer, sondern in mehreren Dimensionen gegenüber dem Fußhalter 20a beweglich ist.

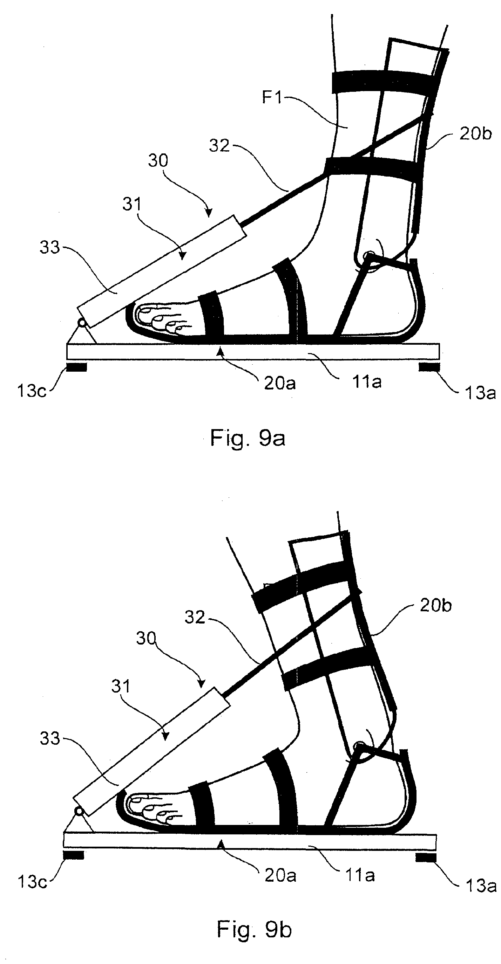

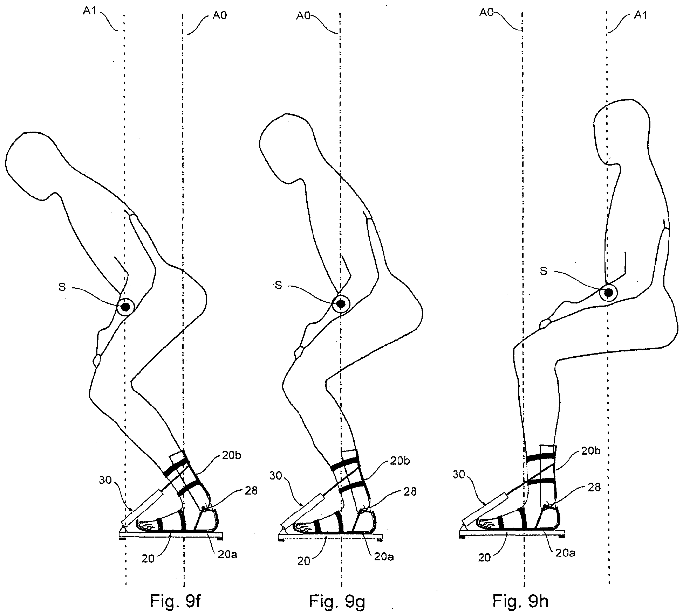

Die Figuren 9a bis 9h zeigen nun eine sechste Ausführungsform der Fixationseinrichtung 20, wobei der wesentliche Unterschied zwischen den vorher beschriebenen Ausführungsformen und derjenigen der vorgenannten Figuren ist, dass eine zusätzliche Krafteinleitung erfolgt. Diese kann sowohl zur Erhöhung der Kraft bzw. des resultierenden Drehmomentes, also zur Erhöhung des Trainingseffektes, als auch zur Entlastung beispielsweise bei nicht selbständig stehfähigen oder muskulär stark geschwächten Anwendern, eingesetzt werden. Hierzu ist eine Krafterzeugungseinrichtung 30 vorgesehen, die im hier gezeigten Fall als eine Kolbenanordnung 31 ausgebildet ist. Eine Kolbenstange 32 der Kolbenanordnung 31 ist an ihrem freien Ende mit der Unterschenkelhalterung 20b verbunden. Ein die Kolbenstange 32 aufnehmender Zylinder 33 ist mit seinem freien Ende beweglich mit der Trittplattform 11a verbunden. Natürlich ist es auch möglich, dass die Trittplattformen 11a, 11b entfallen und dann der Zylinder 33 beweglich mit dem Grundelement 4 der Vorrichtung 1 verbunden ist. Die Kolbenanordnung 31 kann als Druck- oder Zug-Kolbenanordnung 31 ausgebildet sein. Im letztgenannten Fall bewirkt dann die Kolbenanordnung 31, dass der Benutzer zum Vorwärtsbewegen des Unterschenkels die von der Druckkolbenanordnung aufgebrachte Kraft überwinden muss. Bei der Ausbildung der Zylinderanordnung 31 als eine Zugkolbenanordnung bewirkt die auf die Unterschenkelhalterung 20b aufgebrachte Kraft, dass der Unterschenkel des Benutzers durch die Kolbenanordnung 31 nach vorne gezogen wird, so dass der Benutzer für ein Aufrichten aus dieser Lage eine entsprechende Kraft aufwenden muss. Diese Kolbenanordnung kann alternativ auch durch Federn, Gummizüge oder eine einfache Reibkupplung ersetzt werden.

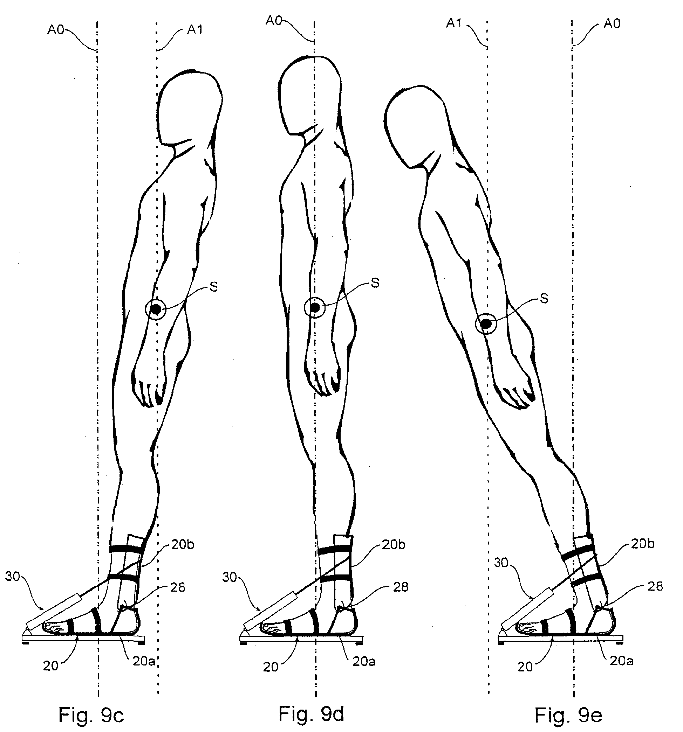

Die Figuren 9c bis 9h zeigen nun die Fixationseinrichtung gemäß den Figuren 9a und 9b auftretenden Drehmomentverhältnisse. In den Figuren 9c bis 9e ist der Benutzer der Vorrichtung 1 stehend dargestellt. Die Darstellung der Figur 9c zeigt einen Benutzer, der sich nach hinten neigt, die der Figur 9d einen gerade stehenden und sich in seiner neutralen Lage befindlichen Benutzer und die Abbildung der Figur 9e einen nach vorne geneigten Benutzer der Vorrichtung 1. Die Achse A0 zeigt die Projektion des Körperschwerpunkts S in Ruhelage und die Achse A1 den resultierenden Auslenkungswinkel in Vor - und Rücklage. Dann befindet sich die Projektion des Körperschwerpunkts S außerhalb der durch die Füße F1, F2 ausgebildeten Unterstützungsfläche des Benutzers. Durch die Fixation der Füße F1, F2 des Benutzers in der Fixationseinrichtung 20 und somit in der Vorrichtung 1 kann sich dieser weiter nach vorne bzw. nach hinten beugen, als dies der Fall wäre, wenn der Benutzer lediglich auf dem Grundelement 4 der Vorrichtung 1 stehen würde. Hierdurch wird das Drehmoment, das der Benutzer durch sein Gewicht aufbringen kann, aufgrund des hierdurch größeren Auslenkungswinkel S erhöht, so dass bereits ohne die oszillierende und/oder vibrierende Bewegung des Grundelements 4 der Vorrichtung 1 ein erhöhtes Drehmoment auf diesen aufgebracht wird, was zu einer deutlichen Erhöhung des Drehmoments im Sprunggelenk führt. Die vorgenannten Bewegungen des Grundelements 4 bewirken dann, dass die vom Benutzer aufzubringende Kraft noch deutlich erhöht wird, so dass ein effektiveres Training des Bewegungsapparats, insbesondere der Heber-Muskulatur der unteren Extremitäten, erreicht wird.

Die Figuren 9f bis 9h zeigen nun den Benutzer der Vorrichtung 1 in einer Hock-Stellung. Man sieht wiederum aus den Figuren, dass die Fixation des Benutzers in der Fixationseinrichtung 20 und somit in der Vorrichtung 1 bereits zu einem deutlich erhöhten Drehmoment führt, da sich der Benutzer - genauso wie bei der stehenden Haltung gemäß der Figuren 9c-9e - weiter in die Vorlage (Figur 9f) oder in die Rücklage (9h) bringen kann, als dies ohne eine derartige Fixation möglich wäre. Die Figuren 9a-9h zeigen daher eine Erhöhung des Drehmoments im Sprunggelenk und zusätzlich in den nachfolgenden Gelenken Knie, Hüfte und Rücken in gebückter Haltung. Die Achse A0 zeigt wiederum die Projektion des Körperschwerpunkts S in der Ausgangsposition in etwa der Mitte der Länge der Füße des Anwenders, Figur 9f eine Vorlage in gebückter Haltung und Figur 9h eine Rücklage in gebückter Haltung. Die Achse A1 zeigt jeweils die Projektion des Körperschwerpunkts S in Vor- und Rücklage. Deutlich ist auch hier zu erkennen, dass die Projektion des Körperschwerpunkts S außerhalb der durch die Füße F1, F2 des Benutzers ausgebildeten Unterstützungsfläche liegt, was durch die Nutzung der Fixationseinrichtung 20 ermöglicht wird. Die Figur 9h veranschaulicht deutlich, dass das durch die Rücklage in gebeugter Haltung erhöhte Drehmoment nicht nur im Sprunggelenk, sondern in diesem Fall auch insbesondere am Kniegelenk angreift. Ein solches Drehmoment im Knie ist unter vergleichbaren Bedingungen ohne Nutzung der Fixationseinrichtung 20 nicht oder nur schwer erreichbar. Somit können durch die Fixationseinrichtung 20 der Vorrichtung 1 deutlich größere Drehmomente und damit deutlich größere muskuläre Kräfte zum Training genutzt werden. Es ist offensichtlich, dass bei körperlich wenig fitten Anwendung der gewünschte Trainingseffekt auch erreichbar ist, ohne dass die Projektion des Körperschwerpunkts S außerhalb der Stützungsflächen durch die Füße F1, F2 liegt. Dies gilt insbesondere bei Patienten mit neurologischen Erkrankungen oder Lähmungen.

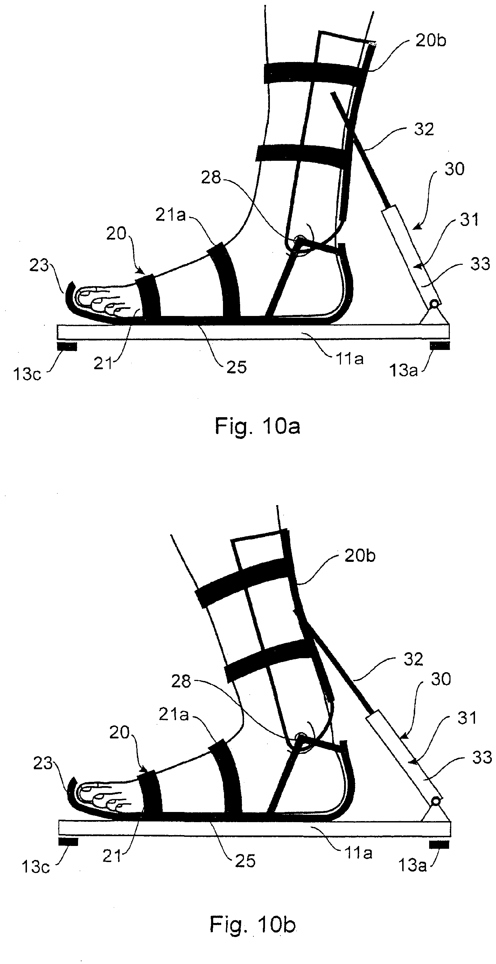

In den Figuren 10a und 10b ist eine siebte Ausführungsform der Fixationseinrichtung 20 dargestellt, die im wesentlichen derjenigen der Figuren 9a und 9b entspricht. Der wesentliche Unterschied zwischen diesen beiden Ausführungsformen ist, dass die Krafterzeugungseinrichtung 30 derart angeordnet ist, dass der Zylinder 33 der Zylinderanordnung 31 nicht - wie bei der Ausgestaltung gemäß der Figuren 9a und 9b - im vorderen Bereich des Grundelements 4 bzw. der Trittplattform 11a bzw. 11b angelenkt ist, sondern hinter dem Fersenbereich. Die sechste und das siebte Ausführungsform der Fixationseinrichtung 20 erlauben es nun, ein um eine zur Fußlängsrichtung quer und durch das Sprunggelenk verlaufenden Achse wirkendes Drehmoment zu erzeugen, indem der Unterschenkel des Benutzers mit der von der Krafterzeugungseinrichtung 30 erzeugten Kraft beaufschlagt wird. Werden die derart ausgebildeten Fixationseinrichtungen 20 zusammen mit der in Figur 1 und 2 gezeigten Vorrichtung 1 verwendet, so ermöglicht dies, die Füße F1, F2 des Benutzers gleichzeitig mit einem durch die Wippbewegung des Grundelements 4 hervorgerufenen, um die Fußlängsachse verlaufenden Drehmoment und gleichzeitig dazu mit einem durch die Krafterzeugungseinrichtung 30 hervorgerufenen, um eine das Sprunggelenk und um eine quer zur Fußlängsachse verlaufenden Drehachse wirkendes Drehmoment zu beaufschlagen. Die da durch ausgebildete Anregungsfunktion für die unteren Extremitäten des Benutzers ist insbesondere für ein Training der Heber-Muskulatur geeignet, da der Benutzer hierbei - gesteuert durch das vegetative Nervensystem - eine komplexe Kompensationsbewegung ausführen muss, um die derart erzeugte Anregungsfunktion durch gegenwirkende Muskelaktionen zu kompensieren.

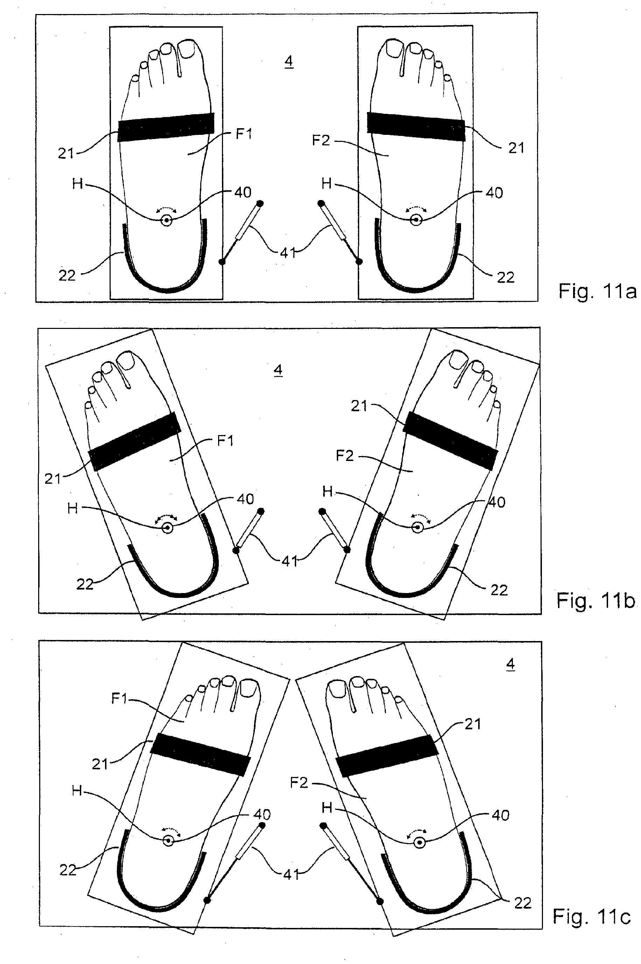

In den Figuren 11a bis 11c ist eine achte Ausführungsform der Fixationseinrichtung 20 dargestellt. Der wesentliche Unterschied zwischen dieser Ausführungsform und den vorstehend beschriebenen Ausführungsformen ist, dass diese eine Bewegung des in der Fixationseinrichtung 20 fixierten Fußes F1 bzw. F2 um eine Hochachse H erlaubt. Hierzu weist die Fixationseinrichtung 20 ein Gelenk 40 auf, um welches sie gegenüber dem Grundelement 4 oder - wenn die Fixationseinrichtung auf einer Trittplattform 11a bzw. 11b befestigt ist - gegenüber dieser um die vorgenannte Hochachse H verschwenkbar ist.

Auch hier kann wieder vorgesehen sein, dass der Benutzer nicht nur mit der vom Grundelement 4 der Vorrichtung 1 erzeugten Anregungsfunktion beaufschlagt wird, sondern dass zusätzlich noch eine entsprechende Krafterzeugungseinrichtung 41 vorgesehen ist, die ihrer Funktion nach der Krafterzeugungseinrichtung 30 der vorstehenden Ausführungsbeispiele entspricht. Dies bedeutet, dass eine Auslenkung des Fußes F1 bzw. F2 aus der durch die Krafterzeugungseinrichtung 41 vorgegebenen Grundstellung einer vom Benutzer aufzubringenden Kraft und/oder eines Drehmoments bedarf. Vorzugsweise ermöglicht die Antriebseinrichtung 41 periodische Anregungen in einem Frequenzbereich zwischen 0,01 Hz und 50 Hz, vorzugsweise zwischen 3 und 35 Hz. Die Amplitude beträgt hierbei vorzugsweise zwischen 1° und 60°.

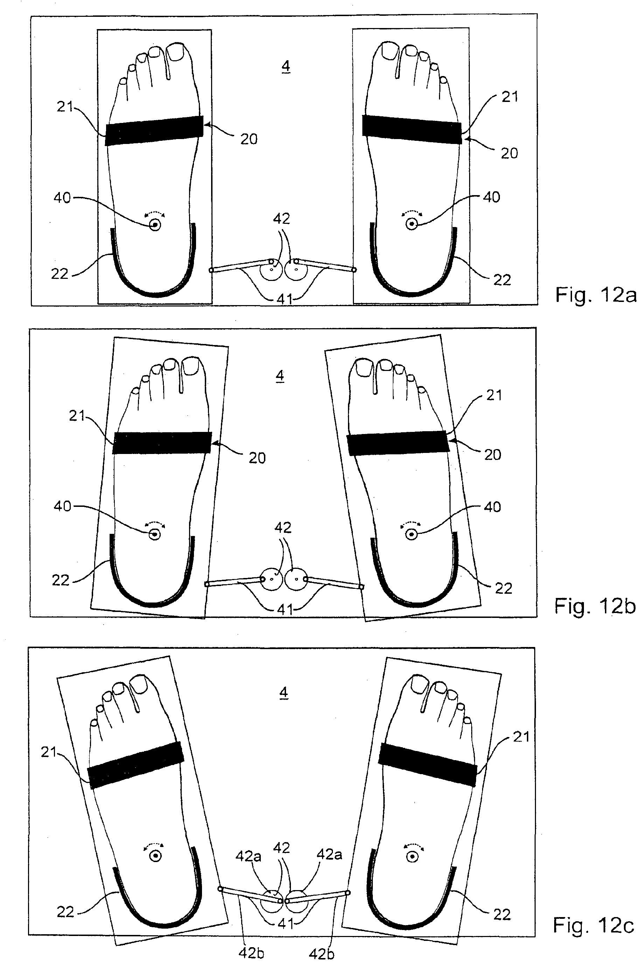

In den Figuren 12a und 12b ist eine neunte Ausführungsform der Fixationseinrichtung 20 dargestellt, die ihrem Grundaufbau nach der achten Ausführungsform der Figuren 10a bis 10c entspricht, so dass einander entsprechende Bauteile mit den gleichen Bezugszeichen versehen und nicht mehr näher beschrieben werden. Die

Krafterzeugungseinrichtung 41 weist einen Antrieb 42 auf, der im hier gezeigten Fall als ein Exzenter-Antrieb mit einer angetrieben Exzenter-Scheibe 42a und einer Exzenterstange 42b, welche die Exzenter-Scheibe 42a mit der die Fixationseinrichtung 20 tragenden Trittplattform 11a, 11b verbindet. Durch eine Rotation der Exzenter-Scheiben 42a wird eine periodische Bewegung der Trittplattformen 11a, 11b und somit der von diesen getragenen Fixationseinrichtung 20 um die Hochachse H erreicht.

Vorstehend wurde davon ausgegangen, dass die Krafterzeugungseinrichtung 41 eine aktive Krafterzeugungseinrichtung ist. Es ist aber auch möglich, dass hier eine passive Krafterzeugungseinrichtung verwendet wird, z. B. ein unter dem Fixationselement 20 angebrachtes Reibband, welches die Reibung zwischen dem Fixationselement 20 und dem dieses tragenden Grundelement 4 bzw. der die Fixationseinrichtung 20 tragenden Trittplattform 11a bzw. 11b erhöht.

Durch Integration eines zusätzlichen elastischen Elementes (nicht gezeigt) zwischen dem Antrieb 42 und der Fixationseinrichtung 20 und und/oder eines weiten Elementes (ebenfalls nicht gezeigt) zur Erzeungung einer Zusatzkraft (z.B. Feder, Gummiband, Pneumatikzylinder) kann eine Vorspannung erreicht werden gegen die der in den Fixationseinrichtungen 20 positionierte Benutzer überlagert zur Schwingung um die Hochachse arbeiten muss. Alternativ kann die Anregung um die Schwingungsanregung um die Hochachse auch entfallen, falls die Anordnung auf eine vorzugsweise vertikal oder seitenalternierend schwingenden Vorrichtung angebracht wird.

Bezüglich der Ausführungsbeispiele der Figuren 11a-11c und 12a-12c ist noch auszuführen, dass natürlich die beschriebene Ausgestaltung mit sämtlichen der vorgenannten Ausführungsformen der Figuren 5 bis 10a-10c kombiniert werden können.

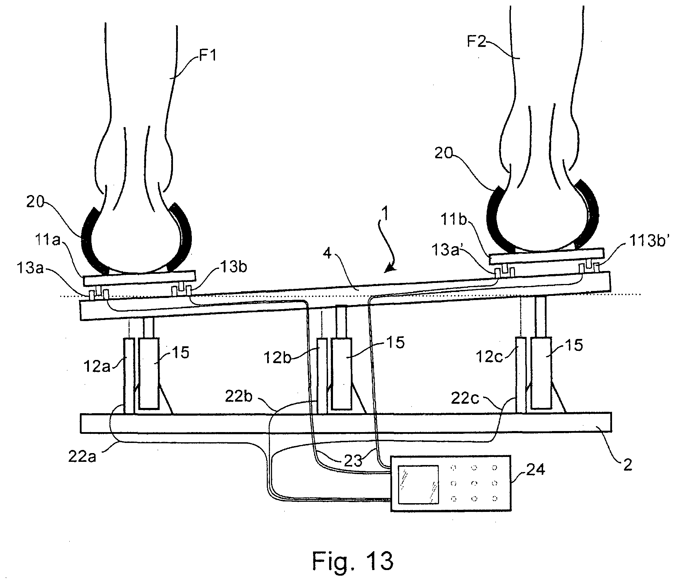

In den Figuren 13 bis 15 ist ein zweites Ausführungsbeispiel einer Vorrichtung 1 dargestellt, wobei wiederum entsprechende Bauteile mit den gleichen Bezugszeichen versehen und nicht mehr näher beschrieben werden. Der wesentliche Unterschied zwischen dem ersten und dem zweiten Ausführungsbeispiel besteht in der Art und Weise, wie das Grundelement 4 in Bewegung versetzt wird, also in der Art und Weise der Erzeugung der Anregungsfunktion. Anstelle des Antriebs des Grundelements 4 über Pleuel 10 ist hier vorgesehen, dass das Grundelement 4 auf drei Linearaktoren 15 angeordnet ist, welche entsprechende Auf- und Abbewegungen durchführen und somit eine Bewegung des Grundelements 4 hervorrufen. Es bedarf keiner weiteren Erläuterung, dass es auch möglich ist, anstelle der drei Linearaktoren 15 nur einen oder zwei Linearaktoren oder mehr als drei Linearaktoren zu verwenden. Eine derartige Ausgestaltung der Antriebseinrichtung 5 der Vorrichtung 1 ist insbesondere für eine aperiodische Anregungsfunktion geeignet, **welche nicht Gegenstand der beanspruchten Erfindung ist, da hierdurch (d. h. durch die vorgenannten Linearaktoren)** zum Beispiel Stöße, Vibrationen, Schwenk-, Kipp oder Drehbewegungen und/oder eine Kombination der vorgenannten Anregungen erzielbar sind. Die Vorrichtung 1 weist hier drei Abstandssensoren 12a-12c auf, mit denen die Lage des Grundelements 4 erfassbar ist. Dem Fachmann ist klar ersichtlich, dass sämtliche der vorstehend beschriebenen Ausführungsformen der Fixationseinrichtung 20 auch bei der Vorrichtung 1 gemäß dem zweiten Ausführungsbeispiel eingesetzt werden können.

Bei der vorstehenden Beschreibung wird davon ausgegangen, dass das Grundelement 4 der Vorrichtung 1 beim Training beide Füße F1, F2 trägt, so dass eine durchgehende Grundplatte als Grundelement 4 der Vorrichtung 1 vorgesehen ist. Dies ist aber nicht zwingend erforderlich.

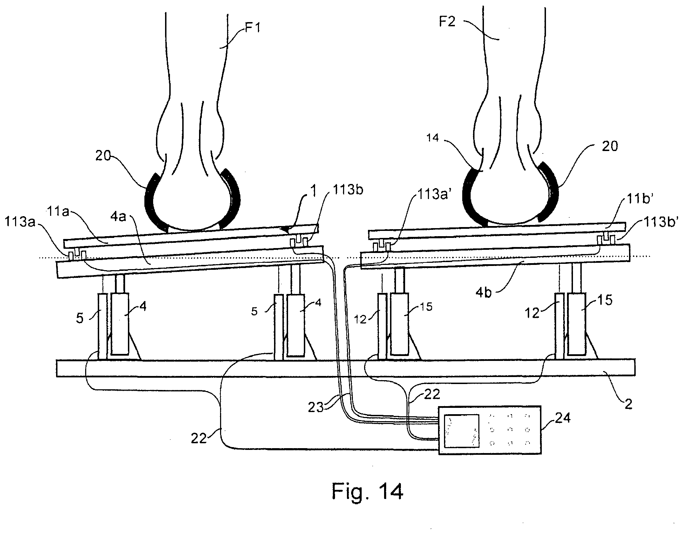

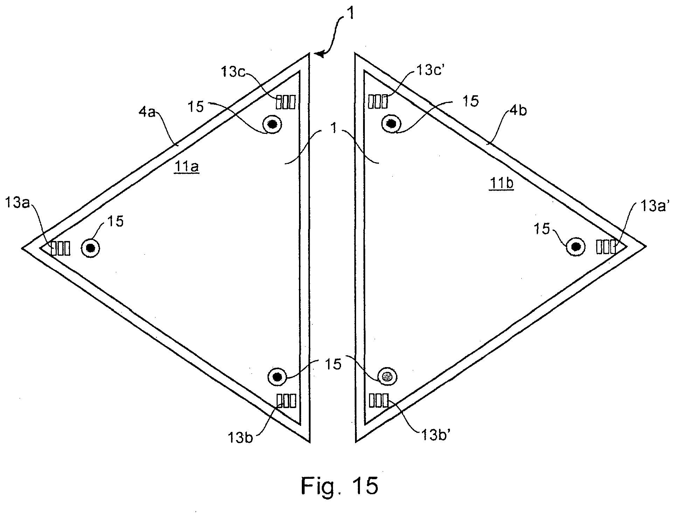

In den Figuren 14 und 15 ist ein drittes Ausführungsbeispiel der Vorrichtung 1 dargestellt, wobei auch hier wiederum einander entsprechende Bauteile mit den gleichen Bezugszeichen versehen und nicht mehr erneut erläutert werden. Der wesentliche Unterschied zu den beiden vorgenannten Ausführungsbeispielen besteht darin, dass das Grundelement 4 zweiteilig ausgeführt ist, dass also hier zwei Teil-Grundelemente 4a und 4b vorhanden sind, die zusammenwirkend die Funktion des Grundelements 4 des ersten Ausführungsbeispiels realisieren. Der Vorteil einer derartigen Konstruktion ist, dass hier die beiden Teil-Grundelemente 4a und 4b unabhängig voneinander bewegt werden können. Die beschriebene Vorrichtung 1 erlaubt es somit in vorteilhafter Art und Weise, den Bewegungsapparat des Benutzers nur einseitig oder seitenspezifisch unterschiedlich stark anzuregen, indem zum Beispiel ein Teil-Grundelement 4a nicht und das zweite Teil-Grundelement 4b wie vorstehend in Schwingungen versetzt wird, oder dass die Amplitude und/oder die Frequenz der Schwingungen der beiden Teil-Grundelemente 4a, 4b unterschiedlich ausgeprägt sind. Insbesondere in diesem Fall ist es dann vorteilhaft, dass die dem jeweiligen Grund-Teilelementen 4a, 4b zugeordneten Sensoren getrennt ausgewertet werden.

Selbstverständlich kann bei dem zweiten und beim dritten Ausführungsbeispiel der Vorrichtung 1 jeder der vorstehend beschriebenen Fixationseinrichtungen 20 verwendet werden.

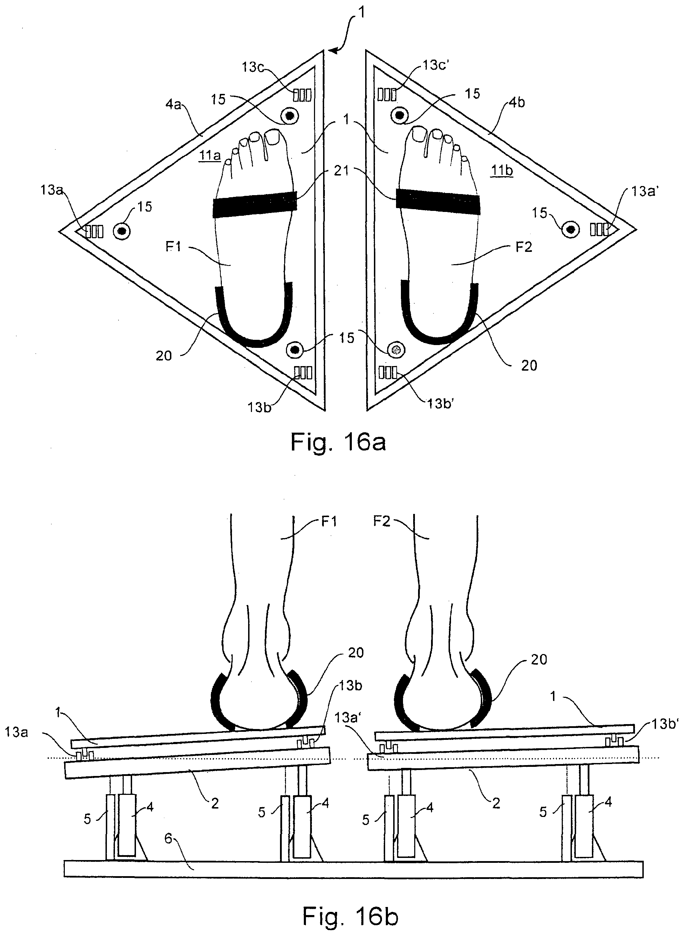

Die Funktionsweise der in den Figuren 14 und 15 dargestellten Vorrichtung 1 wird nun anhand der Figuren 16a und 16b beschrieben, Hierbei wurden die Figuren 14 und 15 dargestellte Vorrichtung 1 nur vereinfacht dargestellt. Durch eine Auslenkung einer oder beider der Teil-Grundelemente 4a, 4b wird wiederum - wie bei dem ersten Ausführungsbeispiel, auf die Figuren 4a bis 4c verwiesen - ein Drehmoment auf den Fuß des Benutzers ausgeübt, wobei die Drehachse wiederum in der Fußlängsrichtung liegt.

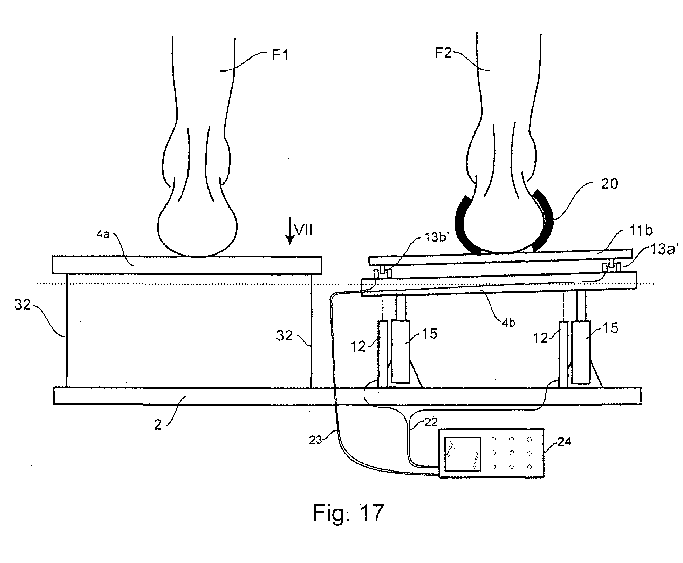

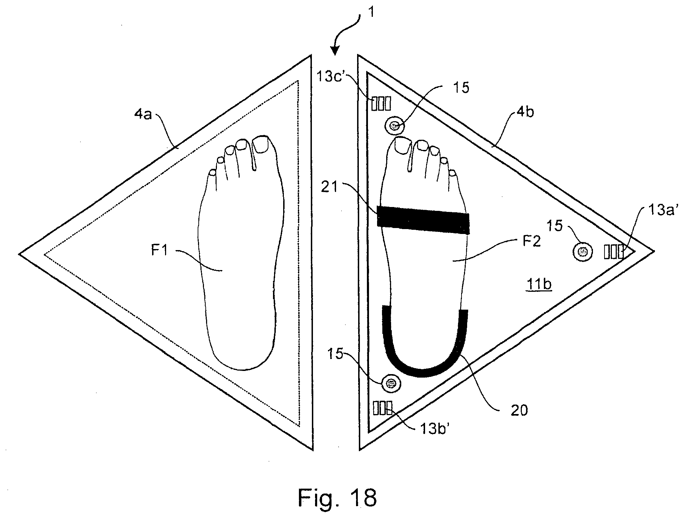

In den Figuren 17 und 18 ist ein viertes Ausführungsbeispiel einer Vorrichtung 1 dargestellt, wobei wiederum entsprechende Teile mit den gleichen Bezugszeichen versehen und nicht mehr näher beschrieben werden. Die Vorrichtung 1 weist wiederum zwei Teil-Grundelemente 4a, 4b auf, wobei aber nur das in Figur rechte Teil-Grundelement 4b in Schwingung versetzbar ist, während das in Figur 16 linke Teil-Grundelement 4a starr ausgebildet ist, indem das erste Teil-Grundelement 4a mit dem Sockel 2 über Trägerelemente 22 fest verbunden ist. Eine derartige Vorrichtung erlaubt in vorteilhafter Art und Weise eine gezielt einseitige Anregung des Bewegungsapparates des Benutzers. Auch hier gilt, dass beim vierten Ausführungsbeispiel der Vorrichtung 1 jeder der vorstehend beschriebenen Fixationseinrichtungen 20 verwendet werden kann.

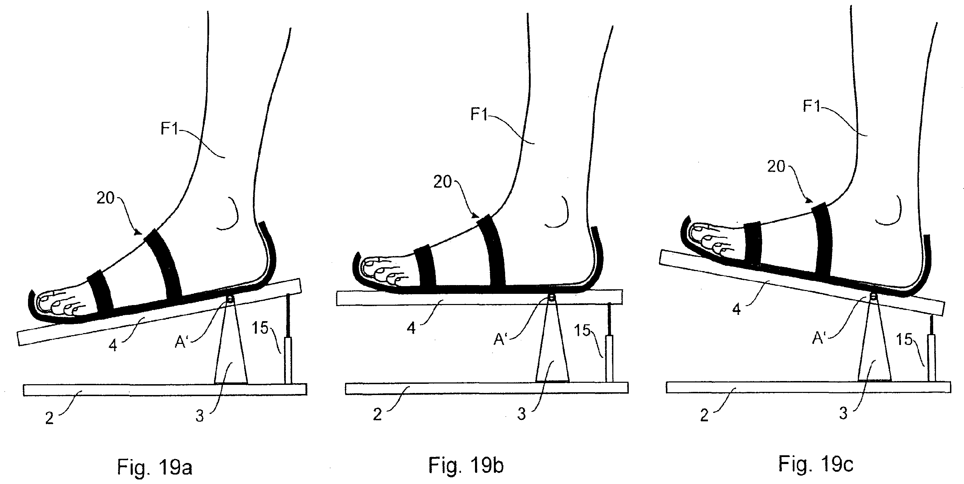

In den Figuren 19a bis 19c ist nun - stark vereinfacht - ein fünftes Ausführungsbeispiel einer Vorrichtung 1 dargestellt, wobei einander entsprechende Bauteile mit den gleichen Bezugszeichen versehen und nicht mehr näher beschrieben werden. Der wesentliche Unterschied zwischen diesem Ausführungsbeispiel und demjenigen der Figuren 1 bis 4, dass das als Wippe fungierende Grundelement 4 anders als beim ersten Ausführungsbeispiel - in einer zur Drehachse A des ersten Ausführungsbeispiel im wesentlichen orthogonal verlaufenden weiteren Drehachse A' verläuft. Die Wippbewegung der Grundplatte 4 bewirkt also nicht mehr, dass - wie beim ersten Ausführungsbeispiel der Vorrichtung 1 - die beiden Füße F1, F2 alternierend auf- und abbewegt werden, sondern, dass die beiden Füße F1, F2 des auf dem Grundelement 4 stehenden Benutzers gleichzeitig um eine um das Sprunggelenk verlaufende Achse aufbewegt werden. Ein weiterer Unterschied zwischen dem ersten und dem hier beschriebenen Ausführungsbeispiel besteht darin, dass das Grundelement 4 nicht - wie beim ersten Ausführungsbeispiel - symmetrisch am Ausleger 3 befestigt ist, sondern wie aus den Figuren ersichtlich - asymmetrisch. Es wird hierbei bevorzugt, dass die Anlenkposition des Grundelements 4 am Ausleger 3 derart gewählt ist, dass die Drehachse A' des Grundelements 4 unterhalb des Sprunggelenks des Fußes F1 liegt. Hierdurch wird eine stabile Position für den Benutzer der Vorrichtung 1 erreicht. Man erkennt aus den vorgenannten Figuren auch, dass im hier gezeigten Fall dieses Grundelement 4 von einem Linearaktuator 15 angetrieben ist, welches dieses in periodische Schwingungen versetzt, denen erforderlichenfalls noch Vibrationen überlagert sind.

Dem Fachmann ist ersichtlich, dass das gleiche Bewegungsmuster mit der Vorrichtung 1 gemäß dem ersten oder dem zweiten Ausführungsbeispiel dadurch erreicht werden kann, dass die Trittplattformen 11a, 11b um 90° verdreht angeordnet werden.

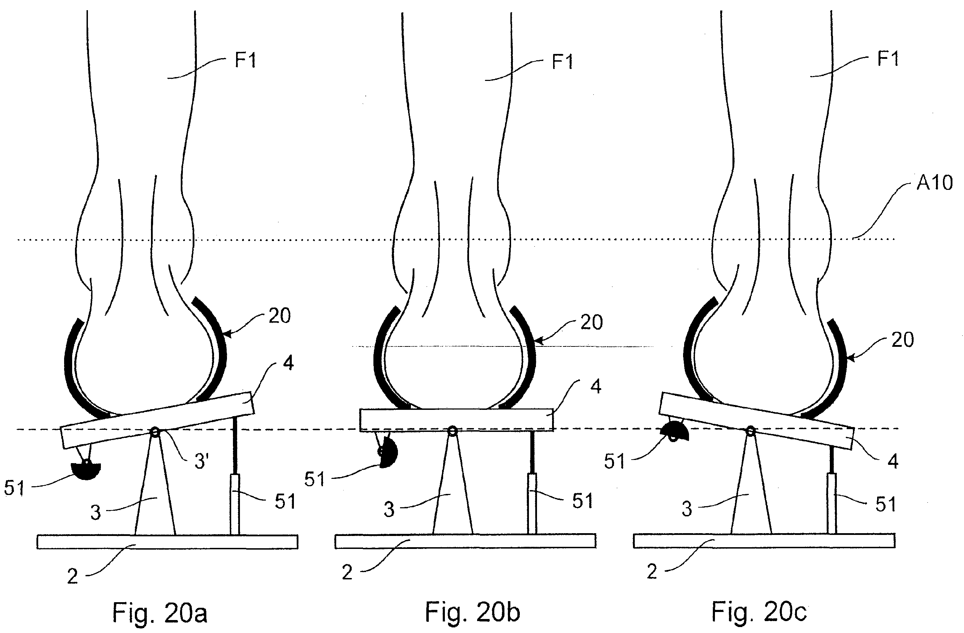

In den Figuren 20a bis 20c ist ein sechstes Ausführungsbeispiel der Vorrichtung 1 dargestellt, wobei der Grundaufbau dieses Ausführungsbeispiels demjenigen der Figuren 17a bis 17c entspricht, mit der Maßgabe, dass hier wiederum das Grundelement 4 symmetrisch am Ausleger 3 angeordnet ist. Man erkennt wiederum, dass durch eine Wippbewegung des Grundelements 4 um den Ausleger 3 der Fuß um eine in Fußlängsrichtung verlaufende Achse um das Sprunggelenk gedreht wird. Der Antrieb des Grundelements 4 erfolgt hierbei durch einen ExzenterAntrieb 50, der seinem Aufbau und seiner Funktion nach demjenigen des ersten Ausführungsbeispiels entspricht. Ein Linearaktuator 51 dient dazu, dass das Grundelement 4 nicht nur die vom Exzenter-Antrieb 50 erzeugte Schwingung durchführt, dass zusätzlich hierzu noch eine Vibration überlagert wird.

Wie aus den Figuren 19a - 19c sowie 20a - 20c ersichtlich ist, erfolgt hierbei die Drehmomentsbeaufschlagung der Füße F1, F2 des Benutzers nicht mehr um eine in Fußlängsrichtung verlaufenden Achse, sondern um eine hierzu im wesentlichen orthogonalen Achse, die wiederum im wesentlichen parallel zur Drehachse A' des Grundelements 4 verläuft. Durch die Bewegung des Grundelements 4 wird somit der Fuß um diese Achse periodisch gedreht und damit ein Drehmoment erzeugt. Die vorstehend genannten beiden Vorrichtungen eignen sich somit besonders zum Training der Heber-Muskulatur des Fußes.

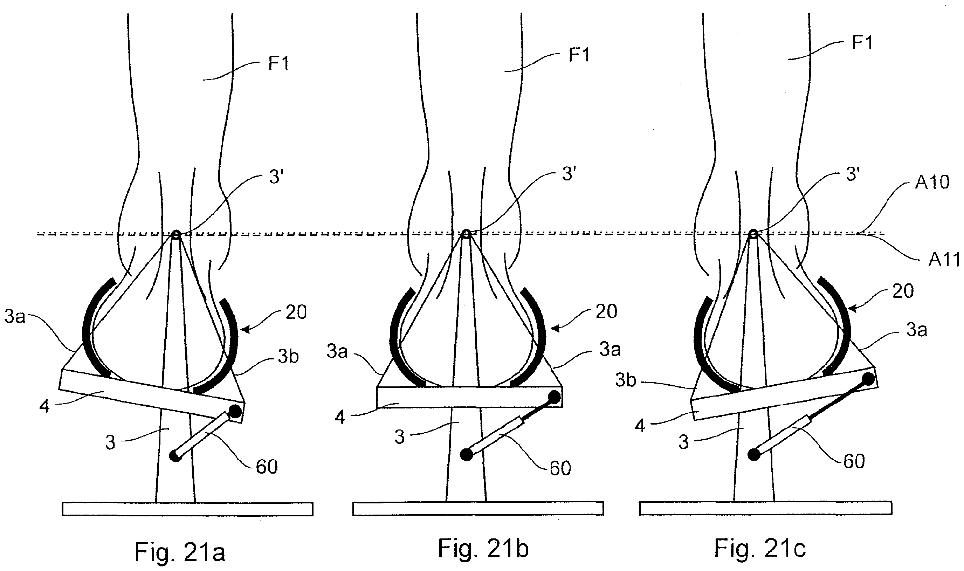

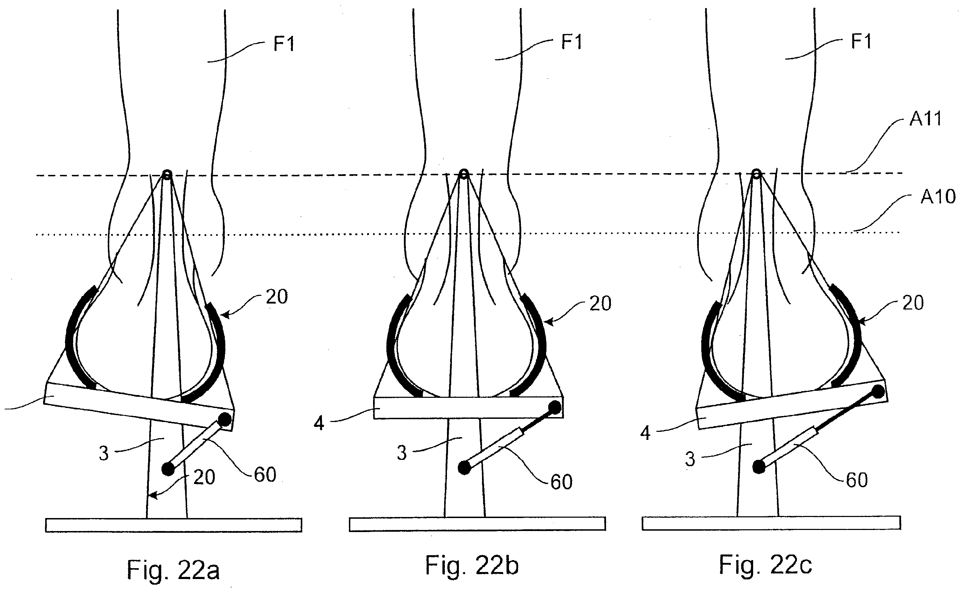

In den Figuren 21a bis 21c sowie 22a bis 22c sind zwei weitere Ausführungsbeispiele der Vorrichtung 1 dargestellt, wobei das Grundelement 4 jeweils schaukelartig am Ausleger 3 befestigt ist, indem Verbindungselemente 3a, 3b vom Anlenkpunkt 3' des Auslegers 3 zu dem Grundelement 4 verlaufen, so dass das Grundelement 4 hierbei eine schwingende Bewegung, angetrieben von der entsprechenden Antriebseinrichtung 60, durchführt. Man erkennt wieder aus den Figuren die Auslenkung des Fußes F1, welche dadurch bewirkt wird, dass der Fuß F1 in der Fixationseinrichtung 20 gehalten am Grundelement 4 lagefixiert ist. Der Unterschied zwischen den beiden Ausführungsformen ist, dass beim Ausführungsbeispiel gemäß den Figuren 21a-21c der Anlenkpunkt 3' des Grundelements 4 am Ausleger 3 über dem Sprunggelenk liegt, während bei dem Ausführungsbeispiel der Figuren 22a-22c dieser Anlenkpunkt im wesentlichen mit der Achse des Sprunggelenks zusammenfällt. In den Figuren ist die Drehachse des Sprunggelenks durch eine punktierte Linie dargestellt, während die Lage des Anlenkpunkts 3' durch eine strichlierte Linie gezeigt ist. Man erkennt aus dieser Figur deutlich die vorstehend beschriebenen Gegebenheiten. Für den Benutzer der Vorrichtung 1 hat dies dann zur Folge, dass sich die Grundplatte 4 der Vorrichtung 1 gemäß den in Figuren 21a-21c in einer stabilen Gleichgewichtslage befindet, während das Grundelement 4 der Vorrichtung gemäß dem Ausführungsbeispiel der Figuren 22a-22c in bezug zum Fuß F1 des Benutzers in einer indifferenten oderwenn das Sprunggelenk höher als der Anlenkpunkt 3' ist - in einer instabilen Lage ist. Die letztbeschriebene Vorrichtung besitzt somit eine größere Kippneigung und ist daher schwieriger zu benutzen als die erstgenannte, was aber auch zur Folge hat, dass hierdurch eine erhöhte Muskelbeanspruchung und somit ein erhöhtes Training der entsprechenden Muskeln verbunden ist.

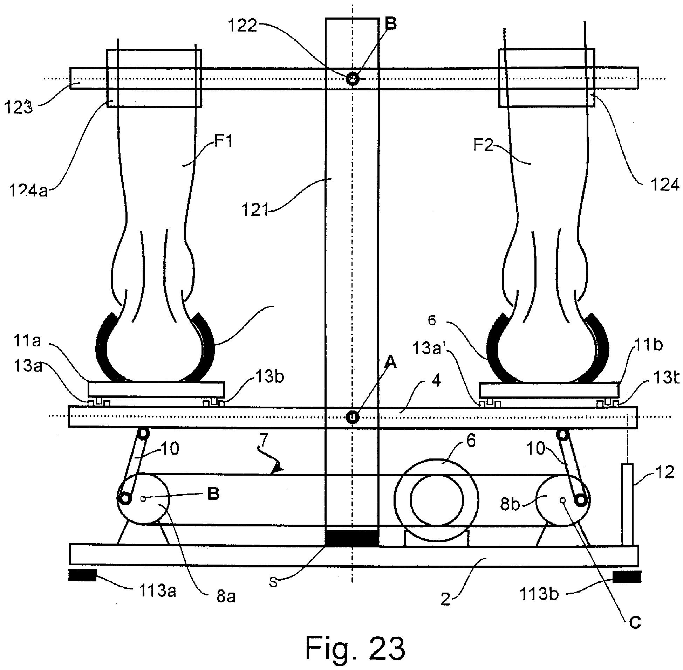

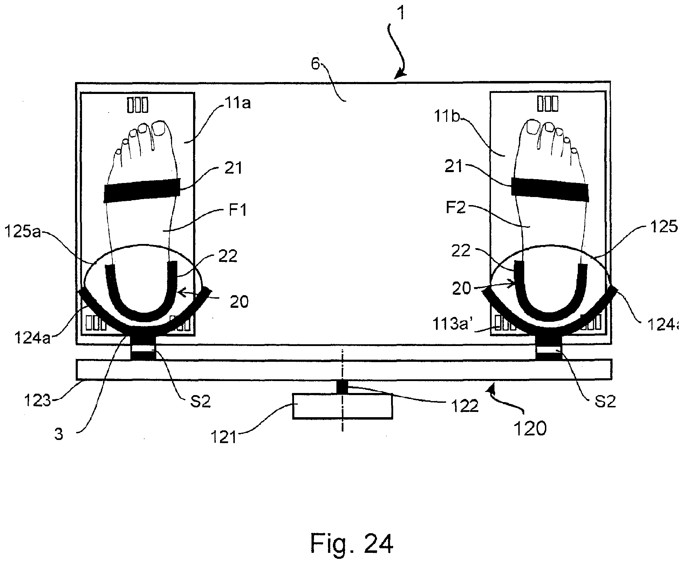

In den Figuren 23 und 24 ist nun ein neuntes Ausführungsbeispiel der Vorrichtung dargestellt, dass seinen Grundaufbau nach dem in den Figuren 1 bis 4 dargestellten ersten Ausführungsbeispiel entspricht. Einander entsprechende Bauteile werden hierbei wieder mit gleichen Bezugszeichen versehen und nicht mehr näher beschrieben. Der wesentliche Unterschied zwischen diesen beiden Ausführungsbeispielen ist nun folgender: Beim ersten Ausführungsbeispiel ist vorgesehen, dass - wie am besten aus den Figuren 3a und 3b ersichtlich - die Fixierung des Fußes F1 bzw. F2 auf der jeweiligen Trittplattform 11a bzw. 11b durch die Schlaufe 21 und den Fersenhalter 22 der dadurch ausgebildeten Fixationseinrichtung 20 erfolgt. Sowohl die Schlaufe 21 als auch der Fersenhalter 22 sind hierbei auf der jeweiligen Trittplattform 11a, 11b befestigt, also auf einem sich während der Benutzung bewegenden Bauteil. Bei dem neunten Ausführungsbeispiel der Vorrichtung 1 ist nun vorgesehen, dass die Fixation der Füße auf den Trittplattformen 11a, 11b nicht mehr (primär) durch jeweils eine auf den Trittplattformen 11a und 11b angeordnete Fersenhalterung 22 durchgeführt wird, sondern dass eine mit dem - unbeweglichen - Sockel 2 der Vorrichtung 1 verbundene, also eine grundplattenexterne Fixationseinrichtung 120 vorgesehen ist, die die Funktion der Fixationseinrichtung 20 übernimmt. Diese weist eine auf dem Sockel 2 aufsetzende Säule 121 auf, mit der über ein Gelenk 122 ein Querträger 123 verbunden ist. An diesem Querträger sind - vorzugsweise gepolsterte - Unterschenkelaufnahmen 124a, 124b vorgesehen. Diese Unterschenkelhalterungen 124a, 124b sind vorzugsweise schalenartig ausgebildet. Optional ist jeweils ein Halteriemen 125 (siehe Figur 24) vorgesehen, mit denen die Unterschenkel des Benutzers in den Unterschenkelhalterungen 124a, 124b fixierbar sind. Das den Querträger 123 mit der Säule 121 verbindende Gelenk 122 ist vorzugsweise auf einem in Längsrichtung der Säule 121 verschiebbaren Lagerung 126 gelagert, so dass der Querträger 123 der über die Unterschenkel des Benutzers übertragenen Vibrationsbewegung der Grundplatte 4 folgen kann. Sind lediglich kleine Vibrationshübe vorgesehen, so kann diese Lagerung 126 entfallen.

Dem Fachmann ist ersichtlich, dass es nicht zwingend ist, dass die Unterschenkelhalterungen 124a, 124b vorhanden sind. Im einfachsten Fall stützt sich der Benutzer der Vorrichtung auf dem Querträger 123 ab. Die vorzugsweise schalenartig ausgebildeten Unterschenkelhalterungen 124a, 124b besitzen aber den Vorteil, dass hierdurch eine Seitenführung der Unterschenkel des Benutzers gewährleistet ist. Dies ist insbesondere dann von Vorteil, wenn Behinderte die Vorrichtung 1 benutzen, die eine seitliche Führung der Unterschenkel im Zweifelsfall nicht selbst bewusst steuern können. Diese Lager 126 sind hierbei entweder passiv ausgeführt, das heißt, sie erlaubt eine Verschiebung des Querträgers 123 infolge der von der Grundplatte 4 erzeugten und über die Beine des Benutzers übertragenen Vibrationen. Es ist aber auch möglich, dass diese Lager 126 aktiv ausgestaltet ist, das heißt, dass sie motorisch angetrieben selbst Vibrationen erzeugt.

Wie aus den Figuren 23 und 24 ersichtlich ist, kann auch bei der Verwendung der Fixationseinrichtung 120 eine Fersenhalterung 22 vorgesehen sein. Dies dient dann zu einer besonders guten Fixierung des Benutzers in der Vorrichtung 1. Es ist aber auch möglich, dass auf diese Fersenhalterung 22 und/oder die Schlaufe 21 verzichtet und die Fixierung des Benutzers in der Vorrichtung 1 nur durch die Fixationseinrichtung 120 bewirkt wird.

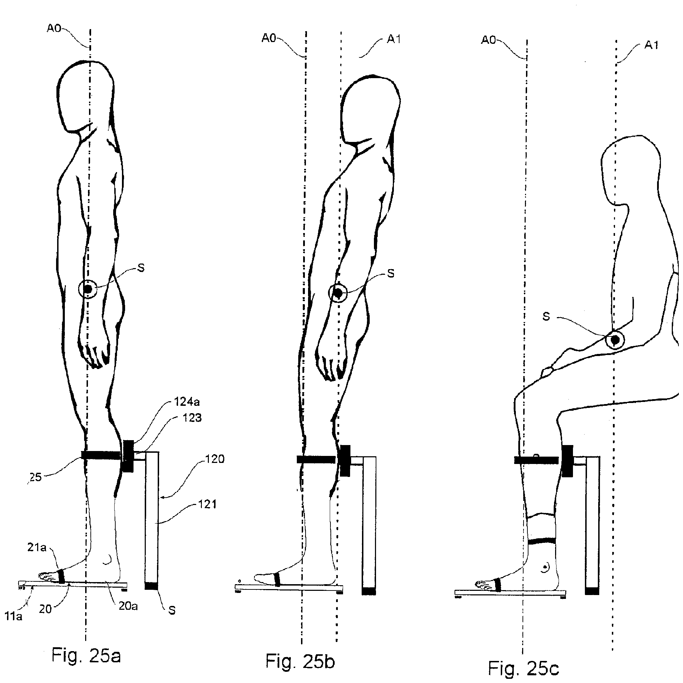

In den Figuren 25a bis 25c ist nun ein zehntes Ausführungsbeispiel der Vorrichtung 1 dargestellt, das eine Fixationseinrichtung 120 verwendet, welche der in Figur 5 dargestellten zweiten Ausführungsform der Fixationseinrichtung 20 entspricht. Einander entsprechende Bauteile werden wieder mit den gleichen Bezugszeichen versehen und nicht mehr näher beschrieben. Der wesentliche Unterschied zwischen der in den Figuren 25a - 25c dargestellten zweiten Ausführungsform der Fixationseinrichtung 120 und der Fixationseinrichtung 20 der Figur 5 ist nun wie folgt: Während bei der Fixationseinrichtung 20 der Figur 5 vorgesehen ist, dass die Fixationseinrichtung 20 Schlaufen 21a, 21b besitzt, die jeweils mit den Trittplattformen 11a bzw. 11b verbunden sind, ist bei der grundplattenexternen Fixationseinrichtung 120 wiederum vorgesehen, dass diese die bereits vorstehend beschriebene Ausbildung mit einer Säule 121 und einem Querträger 123 aufweist, welche nicht auf den Trittplattformen 11a, 11b, sondern auf dem unbeweglichen Sockel 2 der Vorrichtung 1 montiert sind. Die Fixation des Benutzers der Vorrichtung 1 erfolgt dann wieder durch die Fixationseinrichtung 120, so dass im einfachsten Fall die Schlaufen 21, 21a entfallen können. Es ist auch möglich, dass zumindest die Schlaufe 21 verwendet wird, um den Vorderfuß zu fixieren. Natürlich ist es auch möglich, dass eine oder mehrere Schlaufen 21a zusätzlich zu der Fixationseinrichtung 120 verwendet werden.

Um nun zur Durchführung eines Trainings und/oder einer Analyse eines Bewegungsapparates des Benutzers dessen Antwort auf die durch die Vorrichtung 1 aufgebrachte Anregung ermitteln zu können, ist vorgesehen, dass die Vorrichtung 1 einen oder mehrere entsprechend ausgebildete Sensoren aufweist, mit denen die Antwort des Bewegungsapparats des Benutzers auf die Anregung erfassbar ist. Im hier beschriebenen Fall, bei dem auf der Grundplatte Trittplattformen 11a, 11b angeordnet sind, ist vorzugsweise vorgesehen, dass - wie am besten aus Figur 2 ersichtlich ist - im Bereich jeder Trittplattform 11a, 11b drei Sensoren 13a-13c bzw. 13a'-13c' angeordnet sind, welche die vom Benutzer auf die Trittplattformen 11a, 11b und somit auf das Grundelement 4 eingeleiteten Druckkräfte erfassen und deren Sensorsignale über einen Sensorleitung 23 zu der Auswerteeinrichtung 24 geleitet werden. Durch eine kontinuierliche, quasi-kontinuierliche oder zu diskreten Zeitpunkten stattfindende Erfassung und/oder Auswertung der Sensorsignale der Sensoren 13a-13c, 13a'-13c' ist es somit möglich, die vom Benutzer auf das Grundelement 4 ausgeübten Druckkräfte in ihrem zeitlichen Verlauf zu erfassen sowie die daraus ableitbaren physikalischen Größen zu ermitteln. Dadurch ist es möglich, den zeitlichen Verlauf der Antwortfunktion des Benutzers auf die von der Vorrichtung 1 auf ihn ausgeübte Anregungsfunktion zu erfassen.

Die drei Sensoren 13a-13c bzw. 13a'-13c' sind hierbei vorzugsweise nichtkollinear angeordnet, so dass hierdurch nicht nur die vom Benutzer auf die jeweilige Trittplattform 11a, 11b aufgebrachten Druck- und Zugkräfte erfassbar sind, sondern dass aus der räumlichen Beziehung der drei Sensoren 13a-13c bzw. 13a'-13c' der Schwerpunkt der Kraftverteilung und somit der Gesamt-Krafteinleitpunkt ermittelbar ist. Dies erlaubt die genaue Erfassung der Position des Fußes des Benutzers auf der ersten und der zweiten Trittplattform 11a, 11b, woraus wieder die genaue Amplitude der den Fuß beaufschlagende Anregung ermittelbar ist. Es ist aber auch möglich, weniger oder mehr Sensoren zu verwenden oder nur einen einzigen Sensor pro Trittplattform 11a, 11b zu verwenden, der aber dann vorzugsweise auflösend ausgeführt ist. Die Verwendung eines oder mehrerer ortsauflösender Sensoren auf der oder den Trittplattformen 11a, 11b und/oder auf dem Grundelement 4 erlaubt in vorteilhafter Art und Weise eine örtlich aufgelöste Erfassung der vom Benutzer hervorgerufene Druck- oder Kraftverteilung. Wenn auf die Trittplattformen 11a, 11b verzichtet werden soll, so kann dann der entsprechende Bereich des Grundelements 4 mit zur Erfassung der Krafteinleitung geeigneten Sensoren, insbesondere der vorstehend beschriebenen Sensoren, versehen sein.

Ein Vergleich dieser beiden Funktionen erlaubt es nun, die mit der Vorrichtung 1 bewirkte Anregung einerseits und die Reaktion des Benutzers andererseits zueinander in Beziehung zu setzen und durch die Auswertung insbesondere der Amplituden, der Kurvenformen, der Frequenzkomponenten der Kurvenformen und/oder der Phasenlage der beiden Funktionen auf Charakteristika und/oder eine etwaige Fehlfunktion des Bewegungsapparates zu schließen. Die Verwendung der vorgenannten Kraftsensoren 13a-13c bzw. 13a'-13c' erlaubt auch die Bestimmung des Drehmoments: Da die Fußposition und die Sprunggelenksposition auf Grund der Fixationseinrichtung 20 hinreichend bekannt ist, kann aus der Verteilung der Kräfte auf das vom Benutzer erzeugte Drehmoment zurückgeschlossen werden. Beugt sich der Benutzer beispielsweise nach vorne, so muss die Kraft am oder an den oder der vorderen Sensoren 13c, 13c' höher und an den hinteren Sensoren 13a, 13b, 13a', 13b' geringer bzw. negativ (Zug anstelle von Druck) werden. Somit entsteht ein Drehmoment das bei bekannter Geometrie der Anordnung der Sensoren 13a-13c, 13a'-13c' und der Position der Sprunggelenksachse aus dem Körpergewicht und der Kräfte aller Sensoren berechnet werden kann. Bei bekanntem Körpergewicht kann das resultierende Drehmoment auch alleine aus der Geometrie der Sensoranordnung und der auftretenden Kräfte berechnet werden.

Es wird bevorzugt, dass die gemessenen und/oder berechneten Größen mittels visuellem, akustischem und/oder haptischem Signal angezeigt oder derart an den Benutzer der Vorrichtung 1 weitergegeben werden. Eine derartige Maßnahme besitzt den Vorteil, dass mithilfe der vorgenannten Anzeige das Training des Benutzers entsprechend angepasst werden kann.

Vereinfacht kann aber auch ein einzelner Sensor genutzt werden, wenn die Trittfläche - beispielsweise wie in gezeigt - in der Nähe oder vorzugweise in der Sprunggelenksachse gelagert. Aus dem Abstand des Sensors zur Sprunggelenksache und der gemessenen Kraft kann somit direkt das resultierende Moment im Sprunggelenk berechnet werden.

Beim den Ausführungsbeispielen ist die von dem Grundelement 4 auf den Benutzer aufgeprägte Anregungsfunktion eine sinusförmige Funktion, wobei die maximale Auslenkung des in der Figur 1 linken Endes 4' des Grundelements 4 bei einer Phasenlage von 90 ° bzw. 270 ° und die maximale Auslenkung des rechten Endes 4" des Grundelements 4 bei einer Phasenlage von 270 ° bzw. 90 ° stattfindet. Zum Zeitpunkt ihrer maximalen Auslenkung besitzt das Grundelement 4 aber die geringste Geschwindigkeit, welche entsprechend der sinusförmigen Anregung beim Nulldurchgang der Grundplatte 4, also bei einer Phasenlage von 0 ° bzw. 180 °, am größten ist.

Betrachtet man nun den Bewegungsapparat des Benutzers als ein wegerregtes Feder-Masse-Dämpfungssystem, so würde bei einem reinen Feder-Masse-System die Phasenverschiebung zwischen der maximalen Auslenkung der Grundplatte 4 und dem Maximalwert der vom Benutzer auf das Grundelement 4 aufgebrachten Kraft 0 °, bei einem rein dämpfenden System, bei dem die resultierende Kraft von der auf den Benutzer aufgebrachten Geschwindigkeit der Grundplatte 4 abhängt, hingegen 90 ° betragen. Analysiert man nun die Antwortfunktion des Benutzers auf die von der Vorrichtung 1 vorgegebene Anregungsfunktion der Vorrichtung 1 hinsichtlich der Amplitude und/oder der Phasenlage dieser beiden Funktionen, so können in einer dem Fachmann bekannten Art und Weise kinematische Kenngrößen des Bewegungsapparates wie Steifigkeitskoeffizient oder Dämpfungskoeffizient insbesondere im Rahmen des vorgenannten Feder-Masse-Dämpfungs-Systems bestimmt werden.

Von besonderem Vorteil ist, wenn der Auswerteeinrichtung der Vorrichtung das Signal mindestens eines weiteren Sensors zuführbar ist, durch den oder die die Übertragung der Anregungsfunktion von den durch das Grundelement 4, 4a, 4b beaufschlagbaren Extremitäten des Benutzers auf einen weiteren Bereich des Bewegungsapparates erfassbar ist. Es wäre zum Beispiel möglich, Positions-, Weg-, Geschwindigkeits-, oder Beschleunigungssensoren an weiteren Körperteilen des Benutzers, insbesondere am Kopf, anzubringen und die Bewegung des Körperteils, insbesondere der des Kopfes, zu erfassen. Eine möglichst geringe Bewegung des Kopfes weist zum Beispiel auf eine große Elastizität der Wirbelsäule und der Rückenmuskulatur des Benutzers hin, eine starke Bewegung des Kopfes hingegen lässt auf eine geringe Elastizität, die zum Beispiel durch eine Versteifung der Rückenmuskulatur verursacht wird, schließen.

Von weiterem Vorteil ist die Ergänzung der Messeinrichtung durch Sensoren zur Erfassung der Körperhaltung des Benutzers auf der Vorrichtung 1, zum Beispiel durch Verwendung eines ortsauflösenden Kraft- und Drucksensors in optionaler Kombination mit einem Gelenkwinkelsensor, zum Beispiel einem Goniomenter zur Erfassung des Kniewinkels. Auf diese Weise kann durch die Vorrichtung 1 in einfacher Art und Weise ermittelt werden, ob die Kräfte über den Vorfuß oder die Fersen in den Körper eingeleitet werden, was zur Aktivierung unterschiedlicher Muskelgruppen (z. B. vorwiegend Unterschenkel/Wade oder Oberschenkel) führt. Dies kann durch die zusätzliche oder alternative Auswertung des Kniewinkels weiter verfeinert werden. Die vorstehend beschriebenen zusätzlichen sensorischen Informationen erhöhen zum einen den Informationsgehalt der Analyse, zum anderen kann auf deren Basis ein Training auf der Vorrichtung 1 wesentlich besser gesteuert und somit deutlich optimiert werden.

Bei der vorstehenden Beschreibung wurde davon ausgegangen, dass die Sensoren 13a-13c; 13a'-13c' unter den Trittplattformen 11a, 11b angeordnet sind. Es ist aber auch möglich, dass - wie in den Figuren 3a und 3b dargestellt - zusätzlich zu oder anstelle der vorgenannten Sensoren 13a-13c, 13a'-13c' - im hier gezeigten Fall vier - unter dem Sockel 2 angeordnete Sensoren 113a-113d verwendet werden, um die ansonsten von den Sensoren 13a-13c, 13a'-13c' erfassten Parameter zu messen. Die Sensoren 13a-13c, 13a'-13c' können dann entfallen oder die weiteren Sensoren 113a-113d können dann zusätzlich herangezogen werden.

Bei den in den Figuren 23a, 24 und 25a bis 25c dargestellten neunten und zehnten Ausführungsbeispielen, welche die Fixationseinrichtung 120 mit einer Säule 121 und einem Querträger 123 aufweisen, ist es möglich, die Sensorik deutlich einfacher auszugestalten, da hierzu lediglich in die Säule 121 der Fixationseinrichtung 120 ein Drehmoment- und/oder Kraftsensor S eingebaut werden muss. Für den Links-Rechts-Vergleich der Anregung des Benutzers ist es von Vorteil, ergänzend oder alternativ zu dem vorgenannten Sensor S zwei weitere Kraftsensoren S2 zwischen dem Querträger 123 und den entsprechenden Unterschenkelhalterungen 124a, 124b zu verwenden.

## Patentansprüche

1. Vorrichtung für ein Training und/oder eine Analyse eines Bewegungsapparats eines Benutzers, wobei die Vorrichtung (1) mindestens ein Grundelement (4; 4a, 4b), das durch eine Antriebseinrichtung (5; 15; 50; 60) in periodische Bewegungen zur Erzeugung einer auf den Benutzer einwirkenden Anregungsfunktion versetzbar ist, aufweist, wobei das derart angetriebene Grundelement (4) einen oder beide der auf ihm (4) aufsetzenden Füße (F1, F2) in seiner bzw. ihrer Gesamtheit periodisch anhebt und absenkt, wobei die Vorrichtung eine Fixationseinrichtung (20; 120) mit mindestens einem Fußhalter (20) aufweist, durch welche der oder beide Füße (F1, F2) des Benutzers auf dem Grundelement (4) lagefixierbar ist, **dadurch gekennzeichnet, dass** der Fusshalter (20) der Fixationseinrichtung (20;120) derart konfiguriert ist, das der Benutzer während der periodischen Auf- und Abbewegung der Grundplatte (4) der Vorrichtung (1) derart seine Lage verändern kann, dass eine Projektion seines Körperschwerpunktes außerhalb der Unterstützungsfläche des oder der Füße (F1, F2) liegt.

2. Vorrichtung nach Anspruch 1, **dadurch gekennzeichnet, dass** die Fixationseinrichtung (20; 21) der Vorrichtung mindestens einen Unterschenkelhalter (20b; 124a, 124b) aufweist, durch welchen ein Unterschenkel des Benutzers in Bezug auf das Grundelement (4) lagefixierbar ist.

3. Vorrichtung nach Anspruch 1, **dadurch gekennzeichnet, dass** die Fixationseinrichtung (20) auf dem Grundelement (4) der Vorrichtung (1) angeordnet ist.

4. Vorrichtung nach Anspruch 1 oder 3, **dadurch gekennzeichnet, dass** der Fußhalter (20a) der Fixationseinrichtung (20) mindestens ein Befestigungselement (21, 21a-21c) besitzt, mittels dessen der Fuß (F1, F2) des Benutzers in der Fixationseinrichtung (20) befestigbar ist.

5. Vorrichtung nach einem der vorangehenden Ansprüche, **dadurch gekennzeichnet, dass** der Fußhalter (20a) und der Unterschenkelhalter (20b) durch eine Gelenkanordnung (28) miteinander verbunden sind.

6. Vorrichtung nach Anspruch 1, **dadurch gekennzeichnet, dass** die Fixationseinrichtung (20) eine Krafterzeugungseinrichtung (30) besitzt, durch welche auf den Unterschenkel des Benutzers eine Kraft aufbringbar ist, wobei die Krafterzeugungseinrichtung (30) vorzugsweise am Unterschenkelhalter (22b) angreift.

7. Vorrichtung nach einem der vorangehenden Ansprüche, **dadurch gekennzeichnet, dass** die Fixationseinrichtung (20) um eine Hochachse (H) drehbar an dem Grundelement (4) angeordnet ist, und dass vorzugsweise die Vorrichtung eine Krafterzeugungseinrichtung (41) aufweist, durch welche die Fixationseinrichtung (20) um die Hochachse (H) bewegbar ist.

8. Vorrichtung nach einem der vorangehenden Ansprüche, **dadurch gekennzeichnet, dass** zwischen dem Grundelement (4) und der Fixationseinrichtung (20) eine Trittplattform (11a, 11b) angeordnet ist.

9. Vorrichtung nach einem der vorangehenden Ansprüche, **dadurch gekennzeichnet, dass** die Fixationseinrichtung (120) der Vorrichtung (1) grundplattenextern angeordnet ist.

10. Vorrichtung nach Anspruch 9, **dadurch gekennzeichnet, dass** die Fixationseinrichtung (120) auf einem unbeweglichen Sockel (2) der Vorrichtung (1) angeordnet ist, und dass vorzugsweise die Fixationseinrichtung (120) eine auf dem Sockel (2) montierte Säule (121) und einen an dieser angeordneten Querträger (123) aufweist, und dass weiter vorzugsweise der Querträger (123), vorzugsweise über ein Lager (126), beweglich mit der Säule (121) verbunden ist.

11. Vorrichtung nach einem der vorangehenden Ansprüche, **dadurch gekennzeichnet, dass** die Vorrichtung (1) mindestens einen Sensor (13; 13a-13c, 13a'-13c'; 113a-113d; S, S2) zur Ermittlung einer Antwortfunktion eines Benutzers der Vorrichtung (1) auf die durch das Grundelement (4) auf ihn aufgeprägte Anregungsfunktion, dessen oder deren Sensorsignale einer Auswerteeinrichtung (24) der Vorrichtung (1) zugeführt sind, aufweist, und dass durch mindestens einen Sensor (13; 13a-13c, 13a'-13c'; 113a-113d; S, S2) eines resultierenden Drehmoments oder resultierenden Drehmomente ermittelbar ist oder sind, und dass vorzugsweise die Vorrichtung (1) mindestens einen weiteren Sensor (12; 12a-12c) aufweist, durch den die Position und/oder der Bewegungszustand des die Anregungsfunktion der Vorrichtung auf den Benutzer aufprägenden Grundelements (4; 4a, 4b) erfassbar ist und dessen oder deren Sensorsignale der Auswerteeinrichtung (24) zuführbar ist, und dass durch die Auswerteeinrichtung (24) ein Vergleich der durch das Grundelement (4; 4a, 4b) auf den Benutzer aufgeprägten Anregungsfunktion und der Antwortfunktion des Benutzers durchführbar ist, indem die Auswerteeinrichtung (24) die Amplitude und/oder die Phasenlage dieser beiden Funktionen zur Bestimmung von Parametern des Bewegungsapparates des Benutzers vergleicht, und dass die Auswerteeinrichtung (24) das oder die Sensorsignale des oder der weiteren Sensoren (12; 12a-12c) kontinuierlich, quasikontinuierlich oder zu diskreten Zeitpunkten auswertet, und dass weiter vorzugsweise der weitere Sensor (12; 12a-12c) als ein Abstandssensor ausgebildet ist, durch den die Lage des Grundelements (4) erfassbar ist, und/oder als ein Kraft-, Druck-, Weg-, Geschwindigkeits- oder Beschleunigungssensor ausgebildet ist, und dass weiter vorzugsweise der Auswerteeinrichtung (24) der Vorrichtung (1) Sensorsignale mindestens eines weiteren, vorrichtungsexternen Sensors zuführbar sind.

12. Vorrichtung nach einem der vorangehenden Ansprüche, **dadurch gekennzeichnet, dass** auf dem Grundelement (4; 4a, 4b) oder unter der oder mindestens einer der Trittplattformen (11a, 11b) mindestens ein Sensor (13a-13c; 13a'-13c') angeordnet ist, durch den die vom Benutzer auf das Grundelement (4; 4a, 4b) aufgebrachte Kraft und/oder der Druck erfassbar ist.

13. Vorrichtung nach einem der vorangehenden Ansprüche, **dadurch gekennzeichnet, dass** die Fixationseinrichtung (120) mindestens einen Kraft-und/oder Drehmomentsensor (S', S1, S2) besitzt.

14. Vorrichtung nach einem der vorangehenden Ansprüche, **dadurch gekennzeichnet, dass** das Grundelement (4) einteilig ausgebildet ist, oder dass das Grundelement (4) mindestens zwei Teil-Grundelemente (4a, 4b) aufweist.

15. Verfahren für ein Training und/oder eine Analyse eines Bewegungsapparates eines Benutzers, ausgenommen ein Verfahren zur therapeutischen Behandlung des menschlichen oder tierischen Körpers, wobei der Benutzer auf einer Vorrichtung (1) steht, die mindestens ein Grundelement (4; 4a, 4b) aufweist, wobei das Grundelement (4; 4a, 4b) durch eine Antriebseinrichtung (5; 15; 50; 60) zur Erzeugung einer auf den Benutzer einwirkenden Anregungsfunktion in periodische und/oder aperiodische Bewegungen versetzt wird, durch welche ein Fuß (F1, F2) oder beide auf das Grundelement (4) aufsetzenden Füße (F1, F2) des Benutzers in seiner bzw. in ihrer Gesamtheit periodisch und/oder aperiodisch angehoben und abgesenkt werden, wobei der Benutzer durch eine Fixationseinrichtung (20; 120) auf dem Grundelement (4) oder in Bezug auf das Grundelement (4) lagefixiert wird, **dadurch gekennzeichnet, dass** der Fußhalter (20) der Fixationseinrichtung (20; 120) derart konfiguriert ist, dass der Benutzer während der periodischen Auf- und Abbewegung der Grundplatte (4) der Vorrichtung (1) derart seine Lage verändern kann, dass eine Projektion seines Körperschwerpunktes außerhalb der Unterstützungsfläche des oder der Füße (F1, F2) liegt.

## Claims

1. Apparatus for the training and/or analysis of the musculoskeletal system of a user, the apparatus (1) having at least one base element (4; 4a, 4b), which can be put into periodic movements for generating an excitation function acting upon the user, said base element (4) driven in said way moving one or both of the feet (F1, F2) resting on it (4) in its or in their entirety periodically up and down, said apparatus having a fixing device (20; 120) with at least one foot holder (20), by which one foot or both feet (F1, F2) of the user can be fixed in position on the base element (4), **characterized in that** the foot holder (20) of the fixing device (20; 120) is configured such that the user can change his position during the periodic up and down movement of the base element (4) of the apparatus (1) so that a projection of the center of gravity of his body is outside of the support area of the one foot or of both feet (F1, F2).

2. Apparatus according to claim 1, **characterized in that** the fixing device (20; 120) of the apparatus has got at least one lower leg holder (20b; 124a, 124b), by which a lower leg of the user can be held in position on the base element (4).

3. Apparatus according to claim 1, **characterized in that** the fixing device (20) is arranged on the base element (4) of the apparatus (1).

4. Apparatus according to claim 1 or 3, **characterized in that** the foot holder (20a) of the fixing device (20) comprises at least one fastening element (21, 21a-21c), by which the foot (F1, F2) of the user can be attached in the fixing device (20).

5. Apparatus according to one of the previous claims, **characterized in that** the foot holder (20a) and the lower leg holder (20b) are connected via a joint arrangement (28).

6. Apparatus according to claim 1, **characterized in that** the fixing device (20) comprises a force-generating device (30) by means of which a force can be applied on to the lower leg of the user, wherein the force-generating device (30) preferably engages on the lower leg holder (22b).

7. Apparatus according to one of the previous claims, **characterized in that** the fixing device (20) is arranged on the base element (4) movable around a yaw axis (H), and that preferably the apparatus comprises a force-generating device (41), by means of which the fixing device (20) can be moved around the yaw axis (H).

8. Apparatus according to one of the previous claims, **characterized in that** between the base element (4) and the fixing device (20) a step platform (11a, 11b) is provided.

9. Apparatus according to one of the previous claims, **characterized in that** the fixing device (120) of the apparatus (1) is arranged externally to the base plate.

10. Apparatus according to claim 9, **characterized in that** the fixing device (120) is arranged on an immovable base (2) of the apparatus (1), and that preferably the fixing device (120) comprises a column (121) mounted on the base (2) and a cross member (123) arranged on it, and that further preferably the cross member (123) is, preferably via a bearing (126), connected movably with the column (121).

11. Apparatus according to one of the previous claims, **characterized in that** the apparatus (1) comprises at least one sensor (13; 13a-13c, 13a'-13c'; 113a-113d; S, S2) for detecting a response function of the user of the apparatus (1) in response to an excitation function applied to the base element (4) to him, whose sensor signal or whose sensor signals are fed to an evaluation device (24) of the apparatus (1), and that by means of at least one sensor (13; 13a-13c, 13a'-13c'; 113a-113d; S, S2) a resulting torque or resulting torques can be detected, and that preferably the apparatus (1) comprises at least one further sensor (12; 12a-12c), by means of which the position and/or the state of motion of the base element (4; 4a, 4b) applying the excitation function of the apparatus onto the user can be detected and whose sensor signal or whose sensor signals can be led to the evaluation device (24), and that by means of the evaluation device (24) a comparison of the excitation function applied by the base element (4; 4a, 4b) onto the user and the response function of the user can be made **in that** the evaluation device (24) compares the amplitude and/or the phase relation of both of said functions for determining parameters of the musculoskeletal system of the user, and that the evaluation device (24) evaluates the sensor signal or the sensor signals of the one of the sensors (12; 12a-12c) continuously, quasi-continuously or at discrete points in time, and that furthermore preferably the further sensor (12; 12a-12c) is designed as a distance sensor, by which the position of the base element (4) can be detected, and/or is designed as a force, pressure, travel, velocity or acceleration sensor, and that further preferably sensor signals of at least one further sensor not belonging to the apparatus can be led to the evaluation device (24).

12. Apparatus according to one of the previous claims, **characterized in that** on the base element (4; 4a, 4b) or under the or under at least one of the step platforms (11a, 11b) at least one sensor (13a-13c; 13a'-13c') is provided, by means of which the force and/or the pressure applied by the user on the base element (4; 4a, 4b) can be detected.

13. Apparatus according to one of the previous claims, **characterized in that** the fixing device (120) comprises at least one force and/or torque sensor (S', S1, S2).

14. Apparatus according to one of the previous claims, **characterized in that** the base element (4) is formed in one piece, or that the base element (4) comprises at least two part-base elements (4a, 4b).

15. Method for the training and/or the analysis of the musculoskeletal system of a user, with the exception of a method for a therapeutic treatment of a human or an animal body, whereby the user stands on an apparatus (1) having at least one base element (4a; 4a, 4b), whereby the base element (4; 4a, 4b) is put into a periodic movement by means of a driving device (5; 15; 50; 60) for generating an excitation function acting upon the user, through which a foot (F1, F2) or both feet (F1, F2) or the user resting on the base element (4) can be moved in its or in their entirety respectively periodically up and down, whereby the user is held in position by means of a fixing device (20; 120) on the base element (4) or in relation to the base element (4), **characterized in that** the foot holder (20) of the fixing device (20; 120) is configured in such a way that the user can change his position during the periodic up and down movement of the base element (4), so that a projection of his body's center-of-gravity is outside of the support area of the one foot or of both feet (F1, F2).

## Revendications

1. Appareil d'entraînement et/ou d'analyse d'un appareil locomoteur d'un utilisateur, dans lequel l'appareil (1) présente au moins un élément de base (4 ; 4a, 4b) qui peut être déplacé selon des mouvements périodiques grâce à un dispositif d'entraînement (5 ; 15 ; 50 ; 60) afin de générer une fonction d'excitation agissant sur l'utilisateur, dans lequel l'élément de base (4) entraîné de cette manière soulève et abaisse de manière périodique dans son ou leur intégralité un pied ou les deux pieds (F1, F2) reposant sur ledit élément de base (4), dans lequel l'appareil présente un dispositif d'immobilisation (20 ; 120) avec au moins un cale-pied (20) grâce auquel le pied ou les deux pieds (F1, F2) de l'utilisateur peuvent être maintenus en place sur l'élément de base (4), **caractérisé en ce que** le cale-pied (20) du dispositif d'immobilisation (20 ; 120) est configuré de telle sorte que l'utilisateur peut modifier son position pendant le mouvement périodique ascendant et descendant de la plaque de base (4) de l'appareil (1) de telle sorte qu'une projection de son centre de gravité corporel se situe à l'extérieur de la surface d'appui du ou des pieds (F1, F2).

2. Appareil selon la revendication 1, **caractérisé en ce que** le dispositif d'immobilisation (20 ; 21) de l'appareil présente au moins un support de jambe (20b ; 124a, 124b) grâce auquel une jambe de l'utilisateur peut être maintenue en place par rapport à l'élément de base (4).

3. Appareil selon la revendication 1, **caractérisé en ce que** le dispositif d'immobilisation (20) est agencé sur l'élément de base (4) de l'appareil (1).

4. Appareil selon la revendication 1 ou 3, **caractérisé en ce que** le cale-pied (20a) du dispositif d'immobilisation (20) comprend au moins un élément de fixation (21, 21a à 21c) au moyen duquel le pied (F1, F2) de l'utilisateur peut être fixé dans le dispositif d'immobilisation (20).

5. Appareil selon l'une quelconque des revendications précédentes, **caractérisé en ce que** le cale-pied (20a) et le support de jambe (20b) sont reliés l'un à l'autre grâce à un système d'articulation (28).

6. Appareil selon la revendication 1, **caractérisé en ce que** le dispositif d'immobilisation (20) comprend un dispositif de génération de force (30) grâce auquel une force peut être appliquée sur la jambe de l'utilisateur, dans lequel le dispositif de génération de force (30) vient en prise de manière préférée au niveau du support de jambe (22b).

7. Appareil selon l'une quelconque des revendications précédentes, **caractérisé en ce que** le dispositif d'immobilisation (20) est agencé en rotation autour d'un axe vertical (H) sur l'élément de base (4), et **en ce que** l'appareil présente de manière préférée un dispositif de génération de force (41) grâce auquel le dispositif d'immobilisation (20) peut être mis en mouvement autour de l'axe vertical (H).

8. Appareil selon l'une quelconque des revendications précédentes, **caractérisé en ce qu'**un marchepied (11a, 11b) est agencé entre l'élément de base (4) et le dispositif d'immobilisation (20).

9. Appareil selon l'une quelconque des revendications précédentes, **caractérisé en ce que** le dispositif d'immobilisation (120) de l'appareil (1) est agencé de manière externe par rapport à la plaque de base.

10. Appareil selon la revendication 9, **caractérisé en ce que** le dispositif d'immobilisation (120) est agencé sur un socle (2) immobile de l'appareil (1), et **en ce que** le dispositif d'immobilisation (120) présente de manière préférée une colonne (121) montée sur le socle (2) et une traverse (123) agencée au niveau de ladite colonne, et **en ce que** de manière préférée la traverse (123) est en outre reliée de manière mobile à la colonne (121), de manière préférée par l'intermédiaire d'un palier (126).

11. Appareil selon l'une quelconque des revendications précédentes, **caractérisé en ce que** l'appareil (1) présente au moins un capteur (13 ; 13a à 13c, 13a' à 13c' ; 113a à 113d ; S, S2) permettant de déterminer une fonction de réponse d'un utilisateur de l'appareil (1) à la fonction d'excitation qui lui est imposée par l'élément de base (4) et dont les signaux de capteur sont envoyés à un dispositif d'évaluation (24) de l'appareil (1), et **en ce qu'**un couple résultant ou des couples résultants peuvent être déterminés grâce à au moins un capteur (13 ; 13a à 13c, 13a' à 13c' ; 113a à 113d ; S, S2), et **en ce que** de manière préférée l'appareil (1) présente au moins un autre capteur (12 ; 12a à 12c) grâce auquel la position et/ou l'état de mouvement de l'élément de base (4 ; 4a, 4b) imposant à l'utilisateur la fonction d'excitation de l'appareil peuvent être détectés et dont les signaux de capteur peuvent être envoyés au dispositif d'évaluation (24), et **en ce qu'**une comparaison entre la fonction d'excitation imposée à l'utilisateur par l'élément de base (4 ; 4a, 4b) et la fonction de réponse de l'utilisateur peut être mise en œuvre grâce au dispositif d'évaluation (24) étant donné que le dispositif d'évaluation (24) compare l'amplitude et/ou la position de phase desdites deux fonctions afin de déterminer des paramètres de l'appareil locomoteur de l'utilisateur, et **en ce que** le dispositif d'évaluation (24) évalue de manière continue, quasi-continue ou à des instants discrets le signal ou les signaux de capteur de l'autre capteur ou des autres capteurs (12 ; 12a à 12c), et **en ce que** de manière préférée l'autre capteur (12 ; 12a à 12c) est en outre réalisé sous la forme d'un capteur de distance grâce auquel la position de l'élément de base (4) peut être détectée, et/ou sous la forme d'un capteur de force, de pression, de déplacement, de vitesse ou d'accélération, et **en ce que** des signaux de capteur d'au moins un autre capteur externe à l'appareil peuvent en outre être envoyés de manière préférée au dispositif d'évaluation (24) de l'appareil (1).

12. Appareil selon l'une quelconque des revendications précédentes, **caractérisé en ce qu'**au moins un capteur (13a à 13c ; 13a' à 13c'), grâce auquel la force et/ou la pression appliquées par l'utilisateur sur l'élément de base (4 ; 4a, 4b) peuvent être détectées, est agencé sur l'élément de base (4 ; 4a, 4b) ou sous le marchepied ou au moins un des marchepieds (11a, 11b).

13. Appareil selon l'une quelconque des revendications précédentes, **caractérisé en ce que** le dispositif d'immobilisation (120) comprend au moins un capteur de force et/ou de couple (S', S1, S2).

14. Appareil selon l'une quelconque des revendications précédentes, **caractérisé en ce que** l'élément de base (4) est réalisé d'une seule pièce, ou **en ce que** l'élément de base (4) présente au moins deux sous-éléments de base (4a, 4b).

15. Procédé d'entraînement et/ou d'analyse d'un appareil locomoteur d'un utilisateur, à l'exception d'un procédé de traitement thérapeutique du corps humain ou animal, dans lequel l'utilisateur se tient sur un appareil (1) présentant au moins un élément de base (4 ; 4a, 4b), dans lequel, afin de générer une fonction d'excitation agissant sur l'utilisateur, l'élément de base (4 ; 4a, 4b) est déplacé grâce à un dispositif d'entraînement (5 ; 15 ; 50 ; 60) selon des mouvements périodiques grâce auxquels un pied (F1, F2) ou les deux pieds (F1, F2), reposant sur l'élément de base (4), de l'utilisateur sont relevés et abaissés dans son ou leur intégralité de manière périodique, dans lequel l'utilisateur est maintenu en place sur l'élément de base (4) ou par rapport à l'élément de base (4) grâce à un dispositif d'immobilisation (20 ; 120), **caractérisé en ce que** le cale-pied (20) du dispositif d'immobilisation (20 ; 120) est configuré de telle sorte que l'utilisateur peut modifier sa position pendant le mouvement périodique ascendant et descendant de la plaque de base (4) de l'appareil (1) de telle sorte qu'une projection de son centre de gravité corporel se situe à l'extérieur de la surface d'appui du ou des pieds (F1, F2).
